# EUROPEAN PATENT SPECIFICATION

(11) **EP 1 080 095 B1**
(45) Date of publication and mention of the grant of the patent: **02.11.2005**
(21) Application number: 99907332.3
(22) Date of filing: 11.03.1999
(51) Int. Cl.: C07D 513/04, C07D 333/62, C07D 497/04, C07D 209/18, A61K 31/38, A61K 31/395

(54) **MODULATORS OF PROTEIN TYROSINE PHOSPHATASES (PTPASES)**
MODULATOREN DER PROTEIN TYROSIN PHOSPHATASE (PTPASES)
MODULATEURS DE PROTEINE TYROSINE PHOSPHATASES (PTPASES)

(30) Priority: 12.03.1998 DK 34498; 03.04.1998 DK 48098; 15.07.1998 DK 93898; 28.10.1998 DK 138598; 07.12.1998 DK 161298
(43) Date of publication of application: 07.03.2001
(73) Proprietor: NOVO NORDISK A/S, 2880 Bagsvaerd (DK); Ontogen Corporation, Carlsbad, CA 92009 (US)
(72) Inventor: MOLLER, Niels, Peter, Hundahl, DK-2100 Copenhagen (DK); ANDERSEN, Henrik, Sune, DK-2800 Lyngby (DK); IVERSEN, Lars, Fogh, DK-2840 Holte (DK); OLSEN, Ole, Hvilsted, DK-2700 Bronshoj (DK); BRANNER, Sven, DK-2800 Lyngby (DK); HOLSWORTH, Daniel, Dale, San Diego, CA 92129 (US); BAKIR, Farid, San Diego, CA 92120 (US); JUDGE, Luke, Milburn, La Jolla, CA 92037 (US); AXE, Frank, Urban, Escondido, CA 92027 (US); JONES, Todd, Kevin, Solana Beach, CA 92075 (US); RIPKA, Wiliam, Charles, San Diego, CA 92131 (US); GE, Yu, San Diego, CA 92129 (US); UYEDA, Roy, Teruyuki, San Diego, CA 92122 (US)
(74) Representative: Stephensen, Birgitte Borregaard
(86) International application number: PCT/DK1999/000121
(87) International publication number: WO 1999/046267

(56) References cited:
- EP-A1- 0 348 872
- WO-A1-91/16325
- DE-A- 2 509 457
- DE-A1- 3 112 164
- DE-A1- 3 328 438
- US-A- 4 920 043
- US-A- 5 189 054
- CHEMICAL ABSTRACTS, Volume 116, No. 9, 2 March 1992, (Columbus, Ohio, USA), SALITURO FRANCESCO G. et al., "Design, Synthesis and Molecular Modeling of 3-Acylamino-2-Carboxyindole NMDA Receptor Glycine-Site Antagonists", page 800, Abstract No. 83486a; & BIOORG. MED. CHEM. LETT., 1991, 1(9), 455-460.

## Description

### FIELD OF THE INVENTION

The present invention relates to novel compounds and to compositions comprising the compounds, where such compounds of Formula 1 are pharmacologically useful inhibitors of Protein Tyrosine Phosphatases (PTPases) such as PTP1B, CD45, SHP-1, SHP-2, PTPα, LAR and HePTP or the like, 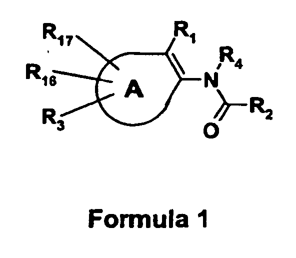 wherein A, R₁, R₂, R₃, R₄, R₁₆ and R₁₇ are defined more fully below.
It has been found that PTPases plays a major role in the intracellular modulation and regulation of fundamental cellular signaling mechanisms involved in metabolism, growth, proliferation and differentiation (Flint et al., The EMBO J. 12:1937-46 (1993); Fischer et al, Science 253:401-6 (1991)). Overexpression or altered activity of tyrosine phosphatases can also contribute to the symptoms and progression of various diseases (Wiener, et al., J. Natl. cancer Inst. 86:372-8 (1994); Hunter and Cooper, Ann. Rev. Biochem, 54:897-930 (1985)). Furthermore, there is increasing evidence which suggests that inhibition of these PTPases may help treat certain types of diseases such as diabetes type I and II, autoimmune disease, acute and chronic inflammation, osteoporosis and various forms of cancer.

### BACKGROUND OF THE INVENTION

Protein phosphorylation is now well recognized as an important mechanism utilized by cells to transduce signals during different stages of cellular function (Fischer et al, Science 253:401-6 (1991); Flint et al., The EMBO J. 12:1937-46 (1993)). There are at least two major classes of phosphatases: (1) those that dephosphorylate proteins (or peptides) that contain a phosphate group(s) on a serine or threonine moiety (termed Ser/Thr phosphatases) and (2) those that remove a phosphate group(s) from the amino acid tyrosine (termed protein tyrosine phosphatases or PTPases).

The PTPases are a family of enzymes that can be classified into two groups: a) intracellular or nontransmembrane PTPases and b) receptor-type or transmembrane PTPases.

*Intracellular PTPases:* Most known intracellular type PTPases contain a single conserved catalytic phosphatase domain consisting of 220-240 amino acid residues. The regions outside the PTPase domains are believed to play important roles in localizing the intracellular PTPases subcellularly (Mauro, L.J. and Dixon, J.E. *TIBS 19*: 151-155 (1994)). The first intracellular PTPase to be purified and characterized was **PTP1B** which was isolated from human placenta (Tonks *et al., J. Biol. Chem. 263*: 6722-6730 (1988)). Shortly after, PTP1B was cloned (Charbonneau *et al., Proc. Natl. Acad. Sci. USA 86:* 5252-5256 (1989); Chernoff *et al., Proc. Natl. Acad. Sci. USA 87*: 2735-2789 (1989)). Other examples of intracellular PTPases include (1) **T-cell PTPase** (Cool *et al. Proc. Natl. Acad. Sci. USA 86*: 5257-5261 (1989)), (2) **rat brain PTPase** (Guan *et al., Proc. Natl. Acad. Sci. USA 87*:1501-1502 (1990)), (3) neuronal phosphatase **STEP** (Lombroso *et al., Proc. Natl. Acad. Sci. USA 88*: 7242-7246 (1991)), (4) ezrin-domain containing PTPases: **PTPMEG1** (Guet *al*., *Proc. Natl. Acad. Sci. USA 88:* 5867-57871 (1991)), **PTPH1** (Yang and Tonks, *Proc. Natl. Acad. Sci. USA 88*: 5949-5953 (1991)), **PTPD1** and **PTPD2** (Møller *et al., Proc. Natl. Acad. Sci. USA 91:* 7477-7481 (1994)), **FAP-1/BAS** (Sato *et al., Science 268*: 411-415 (1995); Banville *et al., J. Biol. Chem. 269*: 22320-22327 (1994); Maekawa *et al., FEBS* *Letters 337*: 200-206 (1994)), and SH2 domain containing PTPases: **PTP1C/SH-PTP1/SHP-1** (Plutzky *et al., Proc. Natl. Acad. Sci. USA 89*: 1123-1127 (1992); Shen *et al., Nature Lond. 352*: 736-739 (1991)) and **PTP1D/Syp/SH-PTP2/SHP-2** (Vogel *et al., Science 259*: 1611-1614 (1993); Feng *et al., Science 259:* 1607-1611 (1993); Bastein *et al., Biochem. Biophys. Res. Comm. 196*: 124-133 (1993)).

Low molecular weight phosphotyrosine-protein phosphatase **(LMW-PTPase)** shows very little sequence identity to the intracellular PTPases described above. However, this enzyme belongs to the PTPase family due to the following characteristics: (i) it possesses the PTPase active site motif: Cys-Xxx-Xxx-Xxx-Xxx-Xxx-Arg (Cirri *et al., Eur. J. Biochem. 214:* 647-657 (1993)); (ii) this Cys residue forms a phospho-intermediate during the catalytic reaction similar to the situation with 'classical' PTPases (Cirri *et al., supra;* Chiarugi *et al., FEBS Lett. 310*: 9-12 (1992)); (iii) the overall folding of the molecule shows a surprising degree of similarity to that of PTP1B and *Yersinia* PTP (Su *et al., Nature 370*: 575-578 (1994)).

*Receptor-type PTPases* consist of a) a putative ligand-binding extracellular domain, b) a transmembrane segment, and c) an intracellular catalytic region. The structures and sizes of the putative ligand-binding extracellular domains of receptor-type PTPases are quite divergent. In contrast, the intracellular catalytic regions of receptor-type PTPases are very homologous to each other and to the intracellular PTPases. Most receptor-type PTPases have two tandemly duplicated catalytic PTPase domains.

The first receptor-type PTPases to be identified were (1) **CD45/LCA** (Ralph, S.J., *EMBO J. 6*: 1251-1257 (1987)) and (2) **LAR** (Streuli *et al., J. Exp. Med. 168:* 1523-1530 (1988)) that were recognized to belong to this class of enzymes based on homology to PTP1B (Charbonneau *et al., Proc. Natl. Acad. Sci. USA 86:* 5252-5256 (1989)). CD45 is a family of high molecular weight glycoproteins and is one of the most abundant leukocyte cell surface glycoproteins and appears to be exclusively expressed upon cells of the hematopoietic system (Trowbridge and Thomas, *Ann. Rev. Immunol. 12*: 85-116 (1994)).

The identification of CD45 and LAR as members of the PTPase family was quickly followed by identification and cloning of several different members of the receptor-type PTPase group. Thus, 5 different PTPases, (3) **PTPα,** (4) **PTPβ,** (5) **PTPδ,** (6) **PTPε,** and (7) **PTPζ,** were identified in one early study (Krueger *et al., EMBO J. 9*: 3241-3252 (1990)). Other examples of receptor-type PTPases include (8) **PTPγ** (Bamea *et al., Mol. Cell. Biol. 13*: 1497-1506 (1995)) which, like **PTPζ** (Krueger and Saito, *Proc. Natl. Acad. Sci. USA 89*: 7417-7421 (1992)) contains a carbonic anhydrase-like domain in the extracellular region, (9) **PTPµ** (Gebbink *et al., **FEBS** Letters 290*: 123-130 (1991)), (10) **PTPκ** (Jiang *et al., Mol. Cell. Biol. 13*: 2942-2951 (1993)). Based on structural differences the receptor-type PTPases may be classified into subtypes (Fischer *et al., Science 253*: 401-406 (1991)): (I) CD45; (II) LAR, PTPd, (11) **PTPσ ;** (III) PTPb, (12) **SAP-1** (Matozaki *et al., J. Biol. Chem. 269*: 2075-2081 (1994)), (13) **PTP-U2/GLEPP1** (Seimiya *et al., Oncogene 10:* 1731-1738 (1995); Thomas *et al., J. Biol. Chem. 269*: 19953-19962 (1994)), and (14) **DEP-1;** (IV) PTPa,_PTPe. All receptor-type PTPases except Type IV contain two PTPase domains. Novel PTPases are continuously identified, and it is anticipated that more than 500 different species will be found in the human genome, i.e. close to the predicted size of the protein tyrosine kinase superfamily (Hanks and Hunter, *FASEB J. 9*: 576-596 (1995)).

PTPases are the biological counterparts to protein tyrosine kinases (PTKs). Therefore, one important function of PTPases is to control, down-regulate, the activity of PTKs. However, a more complex picture of the function of PTPases now emerges. Several studies have shown that some PTPases may actually act as positive mediators of cellular signalling. As an example, the SH2 domain-containing PTP1D seems to act as a positive mediator in insulin-stimulated Ras activation (Noguchi *et al., Mol. Cell. Biol. 14*: 6674-6682 (1994)) and of growth factor-induced mitogenic signal transduction (Xiao *et al., J. Biol. Chem. 269*: 21244-21248 (1994)), whereas the homologous PTP1C seems to act as a negative regulator of growth factor-stimulated proliferation (Bignon and Siminovitch, *Clin.lmmunol. Immunopathol. 73*: 168-179 (1994)). Another example of PTPases as positive regulators has been provided by studies designed to define the activation of the Src-family of tyrosine kinases. In particular, several lines of evidence indicate that CD45 is positively regulating the activation of hematopoietic cells, possibly through dephosphorylation of the C-terminal tyrosine of Fyn and Lck (Chan *et al., Annu. Rev. Immunol. 12*: 555-592 (1994)).

Dual specificity protein tyrosine phosphatases (dsPTPases) define a subclass within the PTPases family that can hydrolyze phosphate from phosphortyrosine as well as from phosphor-serine/threonine. dsPTPases contain the signature sequence of PTPases: His-Cys-Xxx-Xxx-Gly-Xxx-Xxx-Arg. At least three dsPTPases have been shown to dephosphorylate and inactivate extracellular signal-regulated kinase (ERKs)/mitogen-activated protein kinase (MAPK): **MAPK phosphatase** (CL100, 3CH134) (Charles *et al., Proc. Natl. Acad. Sci. USA 90*: 5292-5296 (1993)); **PAC-1** (Ward *et al., Nature 367*: 651-654 (1994)); **rVH6** (Mourey *et al., J. Biol. Chem. 271*: 3795-3802 (1996)). Transcription of dsPTPases are induced by different stimuli, e.g. oxidative stress or heat shock (Ishibashi *et al., J. Biol. Chem. 269*: 29897-29902 (1994); Keyse and Emslie, *Nature 359*: 644-647 (1992)). Further, they may be involved in regulation of the cell cycle: **cdc25** (Millar and Russell, *Cell 68*: 407-410 (1992)); **KAP** (Hannon *et al., Proc. Natl. Acad. Sci. USA 91*: 1731-1735 (1994)). Interestingly, tyrosine dephosphorylation of cdc2 by a dual specific phosphatase, cdc25, is required for induction of mitosis in yeast (review by Walton and Dixon, *Annu. Rev. Biochem. 62*: 101-120 (1993)).

PTPases were originally identified and purified from cell and tissue lysates using a variety of artificial substrates and therefore their natural function of dephosphorylation was not well known. Since tyrosine phosphorylation by tyrosine kinases is usually associated with cell proliferation, cell transformation and cell differentiation, it was assumed that PTPases were also associated with these events.
This association has now been proven to be the case with many PTPases. PTP1B, a phosphatase whose structure was recently elucidated (Barford et al., Science 263:1397-1404 (1994)) has been shown to be involved in insulin-induced oocyte maturation (Flint et al., The EMBO J. 12:1937-46 (1993)) and recently it has been suggested that the overexpression of this enzyme may be involved in p185^{c-erb B2}-associated breast and ovarian cancers (Wiener, et al., J. Natl. cancer Inst. 86:372-8 (1994); Weiner et al., Am. J. Obstet. Gynecol. 170:1177-883 (1994)). The insulin-induced oocyte maturation mechanism has been correlated with the ability of PTP1B to block activation of S6 kinase. The association with cancer is recent evidence which suggests that overexpression of PTP1B is statistically correlated with increased levels of p185^{c-erb B2} in ovarian and breast cancer. The role of PTP1B in the etiology and progression of the disease has not yet been elucidated. Inhibitors of PTP1B may therefore help clarify the role of PTP1B in cancer and in some cases provide therapeutic treatment for certain forms of cancer.

The activity of a number of other newly discussed phosphatases are currently under investigation. Two of these: SHP-1 and Syp/PTP1 D/SHPTP2/PTP2C/SHP-2 have recently been implicated in the activation of Platelet Derived Growth Factor and Epidermal Growth Factor induced responses (Li et al., Mole. Cell. Biol. 14:509-17 (1994)). Since both growth factors are involved in normal cell processing as well as disease states such as cancer and arteriosclerosis, it is hypothesized that inhibitors of these phosphatases would also show therapeutic efficacy. Accordingly, the compounds of the present invention which exhibit inhibitory activity against various PTPases, are indicated in the treatment or management of the foregoing diseases.

### PTPases: the insulin receptor signalling pathway/diabetes

Insulin is an important regulator of different metabolic processes and plays a key role in the control of blood glucose. Defects related to its synthesis or signalling lead to diabetes mellitus. Binding of insulin to its receptor causes rapid (auto)phosphorylation of several tyrosine residues in the intracellular part of the b-subunit. Three closely positioned tyrosine residues (the tyrosine-1150 domain) must all be phosphorylated to obtain full activity of the insulin receptor tyrosine kinase (IRTK) which transmits the signal further downstream by tyrosine phosphorylation of other cellular substrates, including insulin receptor substrate-1 (IRS-1) (Wilden *et al., J. Biol. Chem. 267*: 16660-16668 (1992); Myers and White, *Diabetes 42:* 643-650 (1993); Lee and Pilch, *Am. J. Physiol. 266*: C319-C334 (1994); White *et al., J. Biol. Chem. 263*: 2969-2980 (1988)). The structural basis for the function of the tyrosine-triplet has been provided by recent X-ray crystallographic studies of IRTK that showed tyrosine-1150 to be autoinhibitory in its unphosphorylated state (Hubbard *et al., Nature 372*: 746-754 (1994)).

Several studies clearly indicate that the activity of the auto-phosphorylated IRTK can be reversed by dephosphorylation *in vitro* (reviewed in Goldstein, *Receptor 3:* 1-15 (1993); Mooney and Anderson, *J. Biol. Chem. 264*: 6850-6857 (1989)), with the tri-phosphorylated tyrosine-1150 domain being the most sensitive target for protein-tyrosine phosphatases (PTPases) as compared to the di- and mono-phosphorylated forms (King *et al., Biochem. J.* 275: 413-418 (1991)). It is, therefore, tempting to speculate that this tyrosine-triplet functions as a control switch of IRTK activity. Indeed, the IRTK appears to be tightly regulated by PTP-mediated dephosphorylation *in vivo* (Khan *et al., J. Biol. Chem.* 264: 12931-12940 (1989); Faure *et al., J. Biol. Chem.* 267: 11215-11221 (1992); Rothenberg *et al., J. Biol. Chem. 266:* 8302-8311 (1991)). The intimate coupling of PTPases to the insulin signalling pathway is further evidenced by the finding that insulin differentially regulates PTPase activity in rat hepatoma cells (Meyerovitch *et al., Biochemistry 31:* 10338-10344 (1992)) and in livers from alloxan diabetic rats (Boylan *et al., J. Clin. Invest. 90*: 174-179 (1992)).

Relatively little is known about the identity of the PTPases involved in IRTK regulation. However, the existence of PTPases with activity towards the insulin receptor can be demonstrated as indicated above. Further, when the strong PTPase-inhibitor pervanadate is added to whole cells an almost full insulin response can be obtained in adipocytes (Fantus *et al., Biochemistry 28:* 8864-8871 (1989); Eriksson *et al., Diabetologia 39*: 235-242 (1995)) and skeletal muscle (Leighton *et al., Biochem. J. 276*: 289-292 (1991)). In addition, recent studies show that a new class of peroxovanadiurn compounds act as potent hypoglycemic compounds *in vivo* (Posner *et al.,supra*). Two of these compounds were demonstrated to be more potent inhibitors of dephosphorylation of the insulin receptor than of the EGF-receptor.

It was recently found that the ubiquitously expressed SH2 domain containing PTPase, PTP1D (Vogel *et al.,* 1993, *supra*), associates with and dephosphorylates IRS-1, but apparently not the IR itself (Kuhné *et al., J. Biol. Chem. 268*: 11479-11481 (1993); (Kuhné *et al., J. Biol. Chem. 269*: 15833-15837 (1994)).
Previous studies suggest that the PTPases responsible for IRTK regulation belong to the class of membrane-associated (Faure *et al., J. Biol. Chem. 267*: 11215-11221 (1992)) and glycosylated molecules (Häring *et al., Biochemistry 23*: 3298-3306 (1984); Sale, *Adv. Prot. Phosphatases 6*: 159-186 (1991)). Hashimoto *et al*. have proposed that LAR might play a role in the physiological regulation of insulin receptors in intact cells (Hashimoto *et al., J. Biol. Chem. 267*: 13811-13814 (1992)). Their conclusion was reached by comparing the rate of dephosphorylation/inactivation of purified IR using recombinant PTP1B as well as the cytoplasmic domains of LAR and PTPa. Antisense inhibition was recently used to study the effect of LAR on insulin signalling in a rat hepatoma cell line (Kulas *et al., J. Biol. Chem. 270*: 2435-2438 (1995)). A suppression of LAR protein levels by about 60 percent was paralleled by an approximately 150 percent increase in insulin-induced auto-phosphorylation. However, only a modest 35 percent increase in IRTK activity was observed, whereas the insulin-dependent phosphatidylinositol 3-kinase (PI 3-kinase) activity was significantly increased by 350 percent. Reduced LAR levels did not alter the basal level of IRTK tyrosine phosphorylation or activity. The authors speculate that LAR could specifically dephosphorylate tyrosine residues that are critical for PI 3-kinase activation either on the insulin receptor itself or on a downstream substrate.

While previous reports indicate a role of PTPa in signal transduction through src activation (Zheng *et al., Nature 359:* 336-339 (1992); den Hertog *et al., EMBO J. 12*: 3789-3798 (1993)) and interaction with GRB-2 (den Hertog *et al., EMBO J. 13*: 3020-3032 (1994); Su *et al., J. Biol. Chem. 269*: 18731-18734 (1994)), a recent study suggests a function for this phosphatase and its close relative PTPe as negative regulators of the insulin receptor signal (Møller *et al*., 1995 *supra*). This study also indicates that receptor-like PTPases play a significant role in regulating the IRTK, whereas intracellular PTPases seem to have little, if any, activity towards the insulin receptor. While it appears that the target of the negative regulatory activity of PTPases a and e is the receptor itself, the downmodulating effect of the intracellular TC-PTP seems to be due to a downstream function in the IR-activated signal. Although PTP1B and TC-PTP are closely related, PTP1B had only little influence on the phosphorylation pattern of insulin-treated cells. Both PTPases have distinct structural features that determine their subcellular localization and thereby their access to defined cellular substrates (Frangione *et al., Cell 68:* 545-560 (1992); Faure and Posner, *Glia 9*: 311-314 (1993)). Therefore, the lack of activity of PTP1B and TC-PTP towards the IRTK may, at least in part, be explained by the fact that they do not co-localize with the activated insulin receptor. In support of this view, PTP1B and TC-PTP. have been excluded as candidates for the IR-associated PTPases in hepatocytes based on subcellular localization studies (Faure *et al., J. Biol. Chem.* 267: 11215-11221 (1992)).

The transmembrane PTPase CD45, which is believed to be hematopoietic cell-specific, was in a recent study found to negatively regulate the insulin receptor tyrosine kinase in the human multiple myeloma cell line U266 (Kulas *et al., J. Biol. Chem. 271*: 755-760 (1996)).

### PTPases: somatostatin

Somatostatin inhibits several biological functions including cellular proliferation (Lamberts *et al., Molec. Endocrinol. 8:* 1289-1297 (1994)). While part of the antiproliferative activities of somatostatin are secondary to its inhibition of hormone and growth factor secretion (e.g. growth hormone and epidermal growth factor), other antiproliferative effects of somatostatin are due to a direct effect on the target cells. As an example, somatostatin analogs inhibit the growth of pancreatic cancer presumably via stimulation of a single PTPase, or a subset of PTPases, rather than a general activation of PTPase levels in the cells (Liebow *et al., Proc. Natl. Acad. Sci. USA 86*: 2003-2007 (1989); Colas *et al., Eur. J. Biochem. 207:* 1017-1024 (1992)). In a recent study it was found that somatostatin stimulation of somatostatin receptors SSTR1, but not SSTR2, stably expressed in CHO-K1 cells can stimulate PTPase activity and that this stimulation is pertussis toxin-sensitive. Whether the inhibitory effect of somatostatin on hormone and growth factor secretion is caused by a similar stimulation of PTPase activity in hormone producing cells remains to be determined.

### PTPases: the immune system/autoimmunity

Several studies suggest that the receptor-type PTPase CD45 plays a critical role not only for initiation of T cell activation, but also for maintaining the T cell receptor-mediated signalling cascade. These studies are reviewed in: (Weiss A., *Ann. Rev. Genet. 25*: 487-510 (1991); Chan *et al., Annu. Rev. Immunol. 12*: 555-592 (1994); Trowbridge and Thomas, *Annu. Rev. Immunol. 12:* 85-116 (1994)).
CD45 is one of the most abundant of the cell surface glycoproteins and is expressed exclusively on hemopoetic cells. In T cells, it has been shown that CD45 is one of the critical components of the signal transduction machinery of lymphocytes. In particular, evidence has suggested that CD45 phosphatase plays a pivotal role in antigen-stimulated proliferation of T lymphocytes after an antigen has bound to the T cell receptor (Trowbridge, *Ann. Rev. Immunol,* 12:85-116 (1994)). Several studies suggest that the PTPase activity of CD45 plays a role in the activation of Lck, a lymphocyte-specific member of the Src family protein-tyrosine kinase (Mustelin etal., Proc. Natl. Acad. Sci. USA 86: 6302-6306 (1989); Ostergaard et al., Proc. Natl. Acad. Sci. USA 86: 8959-8963 (1989)). These authors hypothesized that the phosphatase activity of CD45 activates Lck by dephosphorylation of a C-terminal tyrosine residue, which may, in turn, be related to T-cell activation. In a recent study it was found that recombinant p56Ick specifically associates with recombinant CD45 cytoplasmic domain protein, but not to the cytoplasmic domain of the related PTPa (Ng et al., *J. Biol. Chem.* 271: 1295-1300 (1996)). The p56Ick-CD45 interaction seems to be mediated via a nonconventional SH2 domain interaction not requiring phosphotyrosine. In immature B cells, another member of the Src family protein-tyrosine kinases, Fyn, seems to be a selective substrate for CD45 compared to Lck and Syk (Katagiri et al., *J. Biol. Chem.* 270: 27987-27990 (1995)).

Studies using transgenic mice with a mutation for the CD45-exon6 exhibited lacked mature T cells. These mice did not respond to an antigenic challenge with the typical T cell mediated response (Kishihara *et al*., *Cell* 74:143-56 (1993)). Inhibitors of CD45 phosphatase would therefore be very effective therapeutic agents in conditions that are associated with autoimmune disease.

CD45 has also been shown to be essential for the antibody mediated degranulation of mast cells (Berger *et al*., *J. Exp. Med.* 180:471-6 (1994)). These studies were also done with mice that were CD45-deficient. In this case, an IgE-mediated degranulation was demonstrated in wild type but not CD45-deficient T cells from mice. These data suggest that CD45 inhibitors could also play a role in the symptomatic or therapeutic treatment of allergic disorders.

Another recently discovered PTPase, an inducible lymphoid-specific protein tyrosine phosphatase (HePTP) has also been implicated in the immune response. This phosphatase is expressed in both resting T and B lymphocytes, but not non-hemopoetic cells. Upon stimulation of these cells, mRNA levels from the HePTP gene increase 10-15 fold (Zanke *et al., Eur. J. Immunol.* 22:235-239 (1992)). In both T and B cells HePTP may function during sustained stimulation to modulate the immune response through dephosphorylation of specific residues. Its exact role, however remains to be defined.

Likewise, the hematopoietic cell specific PTP1C seems to act as a negative regulator and play an essential role in immune cell development.In accordance with the above-mentioned important function of CD45, HePTP and PTP1C, selective PTPase inhibitors may be attractive drug candidates both as immunosuppressors and as immunostimulants. One recent study illustrates the potential of PTPase inhibitors as immunmodulators by demonstrating the capacity of the vanadium-based PTPase inhibitor, BMLOV, to induce apparent B cell selective apoptosis compared to T cells (Schieven et al., *J. Biol. Chem.* 270: 20824-20831 (1995)).

### PTPases: cell-cell interactions/cancer

Focal adhesion plaques, an *in vitro* phenomenon in which specific contact points are formed when fibroblasts grow on appropriate substrates, seem to mimic, at least in part, cells and their natural surroundings. Several focal adhesion proteins are phosphorylated on tyrosine residues when fibroblasts adhere to and spread on extracellular matrix (Gumbiner, *Neuron 11,* 551-564 (1993)). However, aberrant tyrosine phosphorylation of these proteins can lead to cellular transformation. The intimate association between PTPases and focal adhesions is supported by the finding of several intracellular PTPases with ezrin-like N-terminal domains, e.g. PTPMEG1 (Gu *et al., Proc. Natl. Acad. Sci. USA 88*: 5867-5871 (1991)), PTPH1 (Yang and Tonks, *Proc. Natl. Acad. Sci. USA 88:* 5949-5953 (1991)) and PTPD1 (Moller *et al., Proc. Natl. Acad. Sci. USA 91*: 7477-7481 (1994)). The ezrin-like domain show similarity to several proteins that are believed to act as links between the cell membrane and the cytoskeleton. PTPD1 was found to be phosphorylated by and associated with c-src *in vitro* and is hypothesized to be involved in the regulation of phosphorylation of focal adhesions (Møller *et al., supra*).

PTPases may oppose the action of tyrosine kinases, including those responsible for phosphorylation of focal adhesion proteins, and may therefore function as natural inhibitors of transformation. TC-PTP, and especially the truncated form of this enzyme (Cool *et al., Proc. Natl. Acad. Sci. USA 87*: 7280-7284 (1990)), can inhibit the transforming activity of v-*erb* and v-*fms* (Lammers *et al., J. Biol. Chem. 268:* 22456-22462 (1993); Zander *et al., Oncogene 8:* 1175-1182 (1993)). Moreover, it was found that transformation by the oncogenic form of the *HER2*/*neu* gene was suppressed in NIH 3T3 fribroblasts overexpressing PTP1B (Brown-Shimer *et al., Cancer Res. 52:* 478-482 (1992)).

The expression level of PTP1B was found to be increased in a mammary cell line transformed with *neu* (Zhay *et al., Cancer Res. 53*: 2272-2278 (1993)). The intimate relationship between tyrosine kinases and PTPases in the development of cancer is further evidenced by the recent finding that PTPe is highly expressed in murine mammary tumors in transgenic mice over-expressing c-*neu* and v-Ha-*ras*, but not c-*myc* or *int-2* (Elson and Leder, *J. Biol. Chem. 270:* 26116-26122 (1995)). Further, the human gene encoding PTPg was mapped to 3p21, a chromosomal region which is frequently deleted in renal and lung carcinomas (LaForgia *et al., Proc. Natl. Acad. Sci. USA 88:* 5036-5040 (1991)).

In this context, it seems significant that PTPases appear to be involved in controlling the growth of fibroblasts. In a recent study it was found that Swiss 3T3 cells harvested at high density contain a membrane-associated PTPase whose activity on an average is 8-fold higher than that of cells harvested at low or medium density (Pallen and Tong, *Proc. Natl. Acad. Sci. USA 88*: 6996-7000 (1991)). It was hypothesized by the authors that density-dependent inhibition of cell growth involves the regulated elevation of the activity of the PTPase(s) in question. In accordance with this view, a novel membrane-bound, receptor-type PTPase, DEP-1, showed enhanced (>=10-fold) expression levels with increasing cell density of WI-38 human embryonic lung fibroblasts and in the AG1518 fibroblast cell line (Östman *et al., Proc. Natl. Acad. Sci. USA 91*: 9680-9684 (1994)).

Two closely related receptor-type PTPases, PTPκ and PTPµ, can mediate homophilic cell-cell interaction when expressed in non-adherent insect cells, suggesting that these PTPases might have a normal physiological function in cell-to-cell signalling (Gebbink *et al*., *J. Biol. Chem. 268*: 16101-16104 (1993); Brady-Kalnay *et al*., *J. Cell Biol. 122*: 961-972 (1993); Sap *et al*., *Mol. Cell. Biol. 14*: 1-9 (1994)). Interestingly, PTPk and PTPµ do not interact with each other, despite their structural similarity (Zondag *et al., J. Biol. Chem. 270*: 14247-14250 (1995)). From the studies described above it is apparent that PTPases may play an important role in regulating normal cell growth. However, as pointed out above, recent studies indicate that PTPases may also function as positive mediators of intracellular signalling and thereby induce or enhance mitogenic responses. Increased activity of certain PTPases might therefore result in cellular transformation and tumor formation. Indeed, in one study over-expression of PTPα was found to lead to transformation of rat embryo fibroblasts (Zheng, *supra*). In addition, a novel PTP, SAP-1, was found to be highly expressed in pancreatic and colorectal cancer cells. SAP-1 is mapped to chromosome 19 region q13.4 and might be related to carcinoembryonic antigen mapped to 19q13.2 (Uchida *et al., J. Biol. Chem. 269*: 12220-12228 (1994)). Further, the dsPTPase, cdc25, dephosphorylates cdc2 at Thr14/Tyr-15 and thereby functions as positive regulator of mitosis (reviewed by Hunter, *Cell 80*: 225-236 (1995)). Inhibitors of specific PTPases are therefore likely to be of significant therapeutic value in the treatment of certain forms of cancer.

### PTPases: platelet aggregation

Recent studies indicate that PTPases are centrally involved in platelet aggregation. Agonist-induced platelet activation results in calpain-catalyzed cleavage of PTP1B with a concomitant 2-fold stimulation of PTPase activity (Frangioni *et al., EMBO J. 12*: 4843-4856 (1993)). The cleavage of PTP1B leads to subcellular relocation of the enzyme and correlates with the transition from reversible to irreversible platelet aggregation in platelet-rich plasma. In addition, the SH2 domain containing PTPase, SHP-1, was found to translocate to the cytoskeleton in platelets after thrombin stimulation in an aggregation-dependent manner (Li *et al., FEBS Lett. 343:* 89-93 (1994)).

Although some details in the above two studies were recently questioned there is over-all agreement that PTP1B and SHP-1 play significant functional roles in platelet aggregation (Ezumi *et al., J. Biol. Chem. 270:* 11927-11934 (1995)). In accordance with these observations, treatment of platelets with the PTPase inhibitor pervanadate leads to significant increase in tyrosine phosphorylation, secretion and aggregation (Pumiglia *et al., Biochem. J. 286:* 441-449 (1992)).

### PTPases: osteoporosis

The rate of bone formation is determined by the number and the activity of osteoblasts, which in term are determined by the rate of proliferation and differentiation of osteoblast progenitor cells, respectively. Histomorphometric studies indicate that the osteoblast number is the primary determinant of the rate of bone formation in humans (Gruber *et al., Mineral Electrolyte Metab. 12*: 246-254 (1987); reviewed in Lau *et al., Biochem. J. 257*: 23-36 (1989)). Acid phosphatases/PTPases may be involved in negative regulation of osteoblast proliferation. Thus, fluoride, which has phosphatase inhibitory activity, has been found to increase spinal bone density in osteoporotics by increasing osteoblast proliferation (Lau *et al., supra*). Consistent with this observation, an osteoblastic acid phosphatase with PTPase activity was found to be highly sensitive to mitogenic concentrations of fluoride (Lau *et al., J. Biol. Chem. 260*: 4653-4660 (1985); Lau *et al., J. Biol. Chem.* 262: 1389-1397 (1987); Lau *et al., Adv. Protein Phosphatases 4:* 165-198 (1987)). Interestingly, it was recently found that the level of membrane-bound PTPase activity was increased dramatically when the osteoblast-like cell line UMR 106.06 was grown on collagen type-I matrix compared to uncoated tissue culture plates. Since a significant increase in PTPase activity was observed in density-dependent growth arrested fibroblasts (Pallen and Tong, *Proc. Natl. Acad. Sci. 88*: 6996-7000 (1991)), it might be speculated that the increased PTPase activity directly inhibits cell growth. The mitogenic action of fluoride and other phosphatase inhibitors (molybdate and vanadate) may thus be explained by their inhibition of acid phosphatases/PTPases that negatively regulate the cell proliferation of osteoblasts. The complex nature of the involvement of PTPases in bone formation is further suggested by the recent identification of a novel parathyroid regulated, receptor-like PTPase, OST-PTP, expressed in bone and testis (Mauro *et al., J. Biol. Chem. 269*: 30659-30667 (1994)). OST-PTP is up-regulated following differentiation and matrix formation of primary osteoblasts and subsequently down-regulated in the osteoblasts which are actively mineralizing bone in culture. It may be hypothesized that PTPase inhibitors may prevent differentiation via inhibition of OST-PTP or other PTPases thereby leading to continued proliferation. This would be in agreement with the above-mentioned effects of fluoride and the observation that the tyrosine phosphatase inhibitor orthovanadate appears to enhance osteoblast proliferation and matrix formation (Lau *et al., Endocrinology 116*: 2463-2468 (1988)). In addition, it was recently observed that vanadate, vanadyl and pervanadate all increased the growth of the osteoblast-like cell line UMR106. Vanadyl and pervanadate were stronger stimulators of cell growth than vanadate. Only vanadate was able to regulate the cell differentiation as measured by cell alkaline phosphatase activity (Cortizo *et al., Mol. Cell. Biochem. 145*: 97-102 (1995)).

### PTPases: microorganisms

Dixon and coworkers have called attention to the fact that PTPases may be a key element in the pathogenic properties of *Yersinia* (reviewed in Clemens *et al. Molecular Microbiology 5*: 2617-2620 (1991)). This finding was rather surprising since tyrosine phosphate is thought to be absent in bacteria. The genus *Yersinia* comprises 3 species: *Y. pestis* (responsible for the bubonic plague), *Y. pseudoturberculosis* and *Y. enterocolitica* (causing enteritis and mesenteric lymphadenitis). Interestingly, a dual-specificity phosphatase, VH1, has been identified in Vaccinia virus (Guan *et al., Nature 350*: 359-263 (1991)). These observations indicate that PTPases may play critical roles in microbial and parasitic infections, and they further point to PTPase inhibitors as a novel, putative treatment principle of infectious diseases.

3-Cyano thiophen-2-yl oxamic acid and derivatives thereof are described in DE 2509457 A (The Upjohn Co.). The compounds are stated to be useful for prophylactic treatment of sensitized humans and animals for allergy and all anaphylactic reactions.

Certain 5,6,7,8-tetrahydro-5,8-ethano-pyridino[2,3-b]thieno[5,4-d]pyrimidines are described in US 3,681,351 (Ian Wellings, Wilmington, Delaware). The compounds are stated to have antiviral activity against viruses having a double stranded DNA.

Certain 6-substituted 2-acylamino-4,5,6,7-tetrahydrothieno[2,3-c]pyridine-3-carboxylic acid derivatives are described in DD 272078 A1 (VEB Arzneimittelwerk Dresden). The compounds are stated to be useful for the treatment of allergy.

Modulators of molecules with phosphotyrosine recognition units are described in WO 97/40017 (Novo Nordisk A/S). The modulators are of the general formula (L)ₙ-Ar₁-R₁---A as defined in the application. The compounds are stated to be useful in the treatment of human and animal disorders, in the purification of proteins and glycoproteins and as a diagnostic tool.

### SUMMARY OF THE INVENTION

The present invention relates to compounds of the general formula I, wherein A, R₁, R₂, R₃, R₄, R₁₆ and R₁₇ are as defined in the detailed part of the present description, wherein such compounds are pharmacologically useful inhibitors of Protein Tyrosine Phosphatases (PTPases) such as PTP1B, CD45, SHP-1, SHP-2, PTPα, LAR and HePTP or the like.
The present compounds are useful for the treatment, prevention, elimination, alleviation or amelioration of an indication related to type I diabetes, type II diabetes, impaired glucose tolerance, insulin resistance, obesity, immune dysfunctions including autoimmunity and AIDS, diseases with dysfunctions of the coagulation system, allergic diseases including asthma, osteoporosis, proliferative disorders including cancer and psoriasis, diseases with decreased or increased synthesis or effects of growth hormone, diseases with decreased or increased synthesis of hormones or cytokines that regulate the release of/or response to growth hormone, diseases of the brain including Alzheimer's disease and schizophrenia, and infectious diseases.

In another aspect, the present invention includes within its scope pharmaceutical compositions comprising, as an active ingredient, at least one of the compounds of the general formula I or a pharmaceutically acceptable salt thereof together with a pharmaceutically acceptable carrier or diluent.

### DESCRIPTION OF THE INVENTION

The present invention relates to Compounds of the Formula 1 wherein A, R₁, R₂, R₃, R₄, R₁₆ and R₁₇ are defined below;

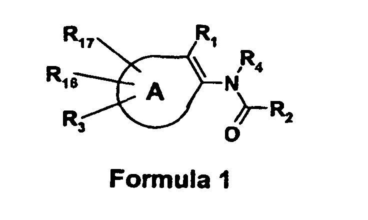

In the above Formula 1
A is together with the double bond in Formula 1 4,5,6,7-tetrahydro-thieno[2,3-*b*]pyridyl, 4,5,6,7-tetrahydro-thieno[2,3-*c*]pyridyl, 4,5,6,7-tetrahydro-thieno[3,2-*c*]pyridyl, 4,5,6,7-tetrahydro-thieno[3,2-*b*]pyridyl, or 4,5,6,7-tetrahydro-4,7-ethano-thieno[2,3-b]pyridyl;
R₁ is hydrogen, COR₅, OR₆, CF₃, nitro, SO₃H, SO₂NR₇R₈, PO(OH)₂, CH₂PO(OH)₂, CHFPO(OH)₂, CF₂PO(OH)₂, C(=NH)NH₂, NR₇R₈ or selected from the following 5-membered heterocycles: 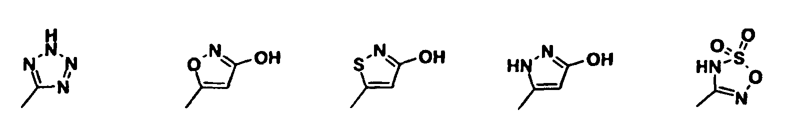 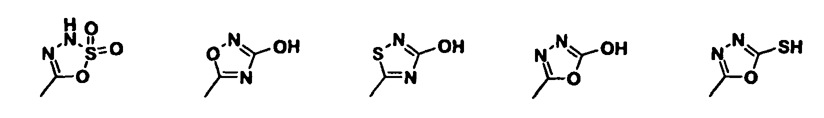 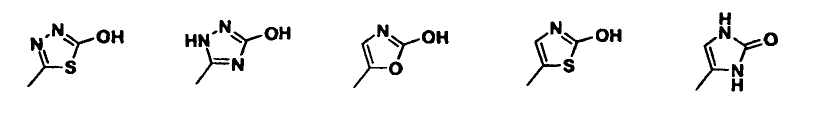 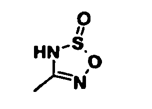 or R₁ is 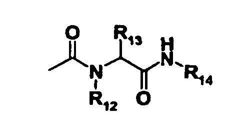 wherein R₁₂, R₁₃, and R₁₄ are independently hydrogen, C₁-C₆alkyl, aryl, arylC₁-C₆alkyl and the alkyl and aryl groups are optionally substituted;
R₂ is COR₅, OR₆, CF₃, nitro, cyano, SO₃H, SO₂NR₇R₈, PO(OH)₂, CH₂PO(OH)₂, CHFPO(OH)₂, CF₂PO(OH)₂, C(=NH)NH₂, NR₇R₈, or selected from the following 5-membered heterocycles: 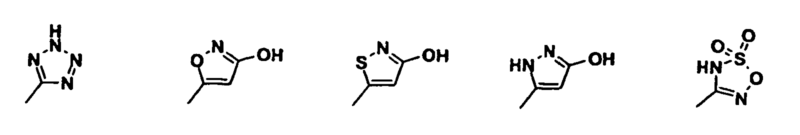 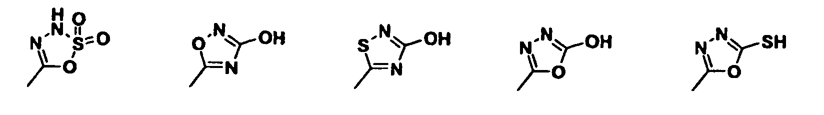 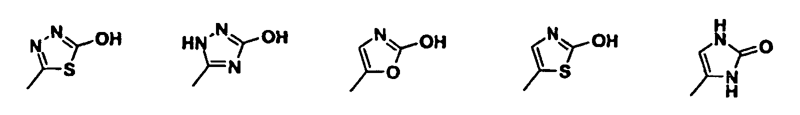 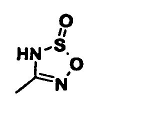
R₃, R₁₆ and R₁₇ are independently hydrogen, halo, nitro, cyano, trihalomethyl, C₁-C₆alkyl, aryl, arylC₁-C₆-alkyl, hydroxy, oxo, carboxy, carboxyC₁-C₆alkyl, C₁-C₆alkyloxycarbonyl, aryloxycarbonyl, arylC₁-C₆alkyloxycarbonyl, C₁-C₆alkyloxy, C₁-C₆alkyloxyC₁-C₆alkyl, aryloxy, arylC₁-C₆alkyloxy, arylC₁-C₆alkyloxyC₁-C₆alkyl, thio, C₁-C₆alkylthio, C₁-C₆alkylthioC₁-C₆alkyl, arylthio, arylC₁-C₆alkylthio, arylC₁-C₆alkylthioC₁-C₆alkyl, NR₇R₈, C₁-C₆alkylaminoC₁-C₆alkyl, arylC₁-C₆alkylaminoC₁-C₆alkyl, di(arylC₁-C₆alkyl)aminoC₁-C₆alkyl, C₁-C₆alkylcarbonyl, C₁-C₆alkylcarbonyl-C₁-C₆alkyl, arylC₁-C₆alkylcarbonyl, arylC₁-C₆alkylcarbonylC₁-C₆alkyl, C₁-C₆alkylcarboxy, C₁-C₆alkylcarboxyC₁-C₆-alkyl, arylcarboxy, arylcarboxyC₁-C₆alkyl, arylC₁-C₆alkylcarboxy, arylC₁-C₆alkylcarboxyC₁-C₆alkyl, C₁-C₆alkylcarbonylamino, C₁-C₆alkylcarbonylaminoC₁-C₆alkyl, -carbonylNR₇C₁-C₆alkylCOR₁₁, arylC₁-C₆alkylcarbonylamino, arylC₁-C₆alkylcarbonylaminoC₁-C₆alkyl, CONR₇R₈, or C₁-C₆alkylCONR₇R₈ wherein the alkyl and aryl groups are optionally substituted and R₁₁ is NR₇R₈, or C₁-C₆alkylNR₇R₈; or R₃ is 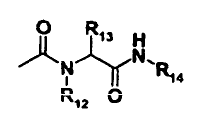 wherein R₁₂, R₁₃, and R₁₄ are independently hydrogen, C₁-C₆alkyl, aryl, arylC₁-C₆alkyl and the alkyl and aryl groups are optionally substituted;
R₄ is hydrogen, hydroxy, C₁-C₆alkyl, aryl, arylC₁-C₆alkyl, NR₇R₈, C₁-C₆alkyloxy; wherein the alkyl and aryl groups are optionally substituted;
R₅ is hydroxy, C₁-C₆alkyl, aryl, arylC₁-C₆alkyl, C₁-C₆alkyl-oxyC₁-C₆alkyloxy, aryloxy, arylC₁-C₆alkyloxy, CF₃; wherein the alkyl and aryl groups are optionally substituted;
R₆ is hydrogen, C₁-C₆alkyl, aryl, arylC₁-C₆alkyl; wherein the alkyl and aryl groups are optionally substituted;
R₇ and R₈ are independently selected from hydrogen, C₁-C₆alkyl, aryl, arylC₁-C₆alkyl, C₁-C₆alkylcarbonyl, arylcarbonyl, arylC₁-C₆alkylcarbonyl, C₁-C₆alkylcarboxy or arylC₁-C₆alkylcarboxy wherein the alkyl and aryl groups are optionally substituted; or R₇ and R₈ are together with the nitrogen to which they are attached forming a saturated, partially saturated or aromatic cyclic, bicyclic or tricyclic ring system containing from 3 to 14 carbon atoms and from 0 to 3 additional heteroatoms selected from nitrogen, oxygen or sulfur, the ring system can optionally be substituted with at least one C₁-C₆alkyl, aryl, arylC₁-C₆alkyl, hydroxy, oxo, C₁-C₆alkyloxy, arylC₁-C₆alkyloxy, C₁-C₆alkyloxyC₁-C₆alkyl, NR₉R₁₀ or C₁-C₆alkylamino-C₁-C₆alkyl, wherein R₉ and R₁₀ are independently selected from hydrogen, C₁-C₆alkyl, aryl, arylC₁-C₆alkyl, C₁-C₆alkylcarbonyl, arylcarbonyl, arylC₁-C₆alkylcarbonyl, C₁-C₆alkylcarboxy or arylC₁-C₆alkylcarboxy; wherein the alkyl and aryl groups are optionally substituted; or R₇ and R₈ are independently a saturated or partial saturated cyclic 5, 6 or 7 membered amine, imide or lactam;
   or a salt thereof with a pharmaceutically acceptable acid or base, or any optical isomer or mixture of optical isomers, including a racemic mixture, or any tautomeric forms;
   wherein alkyl includes C₁-C₆ straight chain saturated, methylene and C₂-C₆ unsaturated aliphatic hydrocarbon groups, C₁-C₆ branched saturated and C₂-C₆ unsaturated aliphatic hydrocarbon groups, C₃-C₆ cyclic saturated and C₅-C₆ unsaturated aliphatic hydrocarbon groups, and C₁-C₆ straight chain or branched saturated and C₂-C₆ straight chain or branched unsaturated aliphatic hydrocarbon groups substituted with C₃-C₆ cyclic saturated and unsaturated aliphatic hydrocarbon groups having the specified number of carbon atoms;
   alkylamino represents one or two alkyl groups as defined above having the indicated number of carbon atoms attached through an amine bridge, wherein the two alkyl groups may be taken together with the nitrogen to which they are attached forming a saturated, partially saturated or aromatic cyclic, bicyclic or tricyclic ring system containing 3 to 14 carbon atoms and 0 to 3 additional heteroatoms selected from nitrogen, oxygen or sulphur;
   aryl represents an unsubstituted, mono-, di- or trisubstituted monocyclic, polycyclic, biaryl and heterocyclic aromatic group covalently attached at any ring position capable of forming a stable covalent bond;
with the proviso that when A together with the double bond in Formula 1 is 4,5,6,7-tetrahydro-thieno[2,3-*c*]pyridyl and R₁ is cyano, then R₂ is not COR₅ wherein R₅ is hydroxy, C₁-C₆-alkyloxy, phenyloxy, or benzyloxy.

### DEFINITIONS

**Signal transduction** is a collective term used to define all cellular processes that follow the activation of a given cell or tissue. Examples of signal transduction, which are not intended to be in any way limiting to the scope of the invention claimed, are cellular events that are induced by polypeptide hormones and growth factors (e.g. insulin, insulin-like growth factors I and II, growth hormone, epidermal growth factor, platelet-derived growth factor), cytokines (e.g. interleukins), extracellular matrix components, and cell-cell interactions.

**Phosphotyrosine recognition units/tyrosine phosphate recognition units/pTyr recognition units** are defined as areas or domains of proteins or glycoproteins that have affinity for molecules containing phosphorylated tyrosine residues (pTyr). Examples of pTyr recognition units, which are not intended to be in any way limiting to the scope of the invention claimed, are: PTPases, SH2 domains and PTB domains.

**PTPases** are defined as enzymes with the capacity to dephosphorylate pTyr-containing proteins or glycoproteins. Examples of PTPases, which are not intended to be in any way limiting to the scope of the invention claimed, are: 'classical' PTPases (intracellular PTPases (e.g. PTP1B, TC-PTP, PTP1C, PTP1D, PTPD1, PTPD2) and receptor-type PTPases (e.g. PTPα, PTPε, PTPβ, PTPγ, CD45, PTPκ, PTPµ), dual specificty phosphatases (VH1, VHR, cdc25), LMW-PTPases or acid phosphatases.

**SH2 domains** (Src homology 2 domains) are non-catalytic protein modules that bind to pTyr (phosphotyrosine residue) containing proteins, i.e. SH2 domains are pTyr recognition units. SH2 domains, which consist of ~100 amino acid residues, are found in a number of different molecules involved in signal transduction processes. The following is a non-limiting list of proteins containing SH2 domains: Src, Hck, Lck, Syk, Zap70, SHP-1, SHP-2, STATs, Grb-2, Shc, p85/PI3K, Gap, vav (see Russell et al, FEBS Lett. 304:15-20 (1992); Pawson, Nature 373: 573-580 (1995); Sawyer, Biopolymers (Peptide Science) 47: 243-261 (1998); and references herein).

As used herein, the term "attached" or "-" (e.g. -COR₁₁ which indicates the carbonyl attachment point to the scaffold) signifies a stable covalent bond, certain preferred points of attachment points being apparent to those skilled in the art.
The terms "halogen" or "halo" include fluorine, chlorine, bromine, and iodine.
The term "alkyl" represents C₁-C₆ straight chain saturated, methylene and C₂-C₆ unsaturated aliphatic hydrocarbon groups, C₁-C₆ branched saturated and C₂-C₆ unsaturated aliphatic hydrocarbon groups, C₃-C₆ cyclic saturated and C₅-C₆ unsaturated aliphatic hydrocarbon groups, and C₁-C₆ straight chain or branched saturated and C₂-C₆ straight chain or branched unsaturated aliphatic hydrocarbon groups substituted with C₃-C₆ cyclic saturated and unsaturated aliphatic hydrocarbon groups having the specified number of carbon atoms. For example, this definition shall include but is not limited to methyl (Me), ethyl (Et), propyl (Pr), butyl (Bu), pentyl, hexyl, heptyl, ethenyl, propenyl, butenyl, penentyl, hexenyl, isopropyl (i-Pr), isobutyl (i-Bu), *tert*-butyl (*t*-Bu), *sec*-butyl (*s*-Bu), isopentyl, neopentyl, cyclopropyl, cyclobutyl, cyclopentyl, cyclohexyl, cyclopentenyl, cyclohexenyl, methylcyclopropyl, ethylcyclohexenyl, butenylcyclopentyl, and the like.
The term "substituted alkyl" represents an alkyl group as defined above wherein the substitutents are independently selected from halo, cyano, nitro, trihalomethyl, carbamoyl, hydroxy, oxo, COR₅, C₁-C₆alkyl, C₁-C₆alkyloxy, aryloxy, arylC₁-C₆alkyloxy, thio, C₁-C₆alkylthio, arylthio, arylC₁-C₆alkylthio, NR₇R₈, C₁-C₆alkylamino, arylamino, arylC₁-C₆alkylamino, di(arylC₁-C₆alkyl)amino, C₁-C₆alkylcarbonyl, arylC₁-C₆alkylcarbonyl, C₁-C₆alkylcarboxy, arylcarboxy, arylC₁-C₆alkylcarboxy, C₁-C₆alkylcarbonyl-amino, -C₁-C₆alkylaminoCOR₁₁, arylC₁-C₆alkylcarbonylamino, tetrahydrofuranyl, morpholinyl, piperazinyl, -CONR₇R₈, -C₁-C₆alkyl-CONR₇R₈, or a saturated or partial saturated cyclic 5, 6 or 7 membered amine, imide or lactam; wherein R₁₁ is hydroxy, C₁-C₆alkyl, aryl, arylC₁-C₆alkyl, C₁-C₆alkyloxy, aryloxy, arylC₁-C₆alkyloxy and R₅ is defined as above or NR₇R₈. wherein R₇, R₈ are defined as above.

The term "saturated, partially saturated or aromatic cyclic, bicyclic or tricyclic ring system" represents aziridinyl, pyrrolyl, pyrrolinyl, pyrrolidinyl, imidazolyl, 2-imidazolinyl, imidazolidinyl, pyrazolyl, 2-pyrazolinyl, 1,2,3-triazolyl, 1,2,4-triazolyl, morpholinyl, piperidinyl, thiomorpholinyl, piperazinyl, indolyl, isoindolyl, 1,2,3,4-tetrahydro-quinolinyl, 1,2,3,4-tetrahydro-isoquinolinyl, 1,2,3,4-tetrahydro-quinoxalinyl, indolinyl, indazolyl, benzimidazolyl, benzotriazolyl, purinyl, carbazolyl, acridinyl, phenothiazinyl, phenoxazinyl, iminodibenzyl, iminostilbenyl.
The term "alkyloxy" (e.g. methoxy, ethoxy, propyloxy, allyloxy, cyclohexyloxy) represents an "alkyl" group as defined above having the indicated number of carbon atoms attached through an oxygen bridge. The term "alkyloxyalkyl" represents an "alkyloxy" group attached through an alkyl group as defined above having the indicated number of carbon atoms.
The term "alkyloxyalkyloxy" represents an "alkyloxyalkyl" group attached through an oxygen atom as defined above having the indicated number of carbon atoms.

The term "aryloxy" (e.g. phenoxy, naphthyloxy and the like) represents an aryl group as defined below attached through an oxygen bridge.
The term "arylalkyloxy" (e.g. phenethyloxy, naphthylmethyloxy and the like) represents an "arylalkyl" group as defined below attached through an oxygen bridge.
The term "arylalkyloxyalkyl" represents an "arylalkyloxy" group as defined above attached through an "alkyl" group defined above having the indicated number of carbon atoms.
The term "arylthio" (e.g. phenylthio, naphthylthio and the like) represents an "aryl" group as defined below attached through an sulfur bridge.
The term "alkyloxycarbonyl" (e.g. methylformiat, ethylformiat and the like) represents an "alkyloxy" group as defined above attached through a carbonyl group.

The term "aryloxycarbonyl" (e.g. phenylformiat, 2-thiazolylformiat and the like) represents an "aryloxy" group as defined above attached through a carbonyl group.
The term "arylalkyloxycarbonyl" (e.g. benzylformiat, phenyletylformiat and the like) represents an "arylalkyloxy" group as defined above attached through a carbonyl group.
The term "alkyloxycarbonylalkyl" represents an "alkyloxycarbonyl" group as defined above attached through an "alkyl" group as defined above having the indicated number of carbon atoms.
The term "arylalkyloxycarbonylalkyl" represents an "arylalkyloxycarbonyl" group as defined above attached through an "alkyl" group as defined above having the indicated number of carbon atoms.
The term "alkylthio" (e.g. methylthio, ethylthio, propylthio, cyclohexenylthio and the like) represents an "alkyl" group as defined above having the indicated number of carbon atoms attached through a sulfur bridge.
The term "arylalkylthio" (e.g. phenylmethylthio, phenylethylthio, and the like) represents an "arylalkyl" group as defined above having the indicated number of carbon atoms attached through a sulfur bridge.
The term "alkylthloalkyl" represents an "alkylthio" group attached through an alkyl group as defined above having the indicated number of carbon atoms.
The term "arylalkylthioalkyl" represents an "arylalkylthio" group attached through an alkyl group as defined above having the indicated number of carbon atoms.

The term "alkylamino" (e.g. methylamino, diethylamino, butylamino, N-propyl-N-hexylamino, (2-cyclopentyl)propylamino, hexenylamino, pyrrolidinyl, piperidinyl and the like) represents one or two "alkyl" groups as defined above having the indicated number of carbon atoms attached through an amine bridge. The two alkyl groups may be taken together with the nitrogen to which they are attached forming a saturated, partially saturated or aromatic cyclic, bicyclic or tricyclic ring system containing 3 to 14 carbon atoms and 0 to 3 additional heteroatoms selected from nitrogen, oxygen or sulfur, the ring system can optionally be substituted with at least one C₁-C₆alkyl, aryl, arylC₁-C₆alkyl, hydroxy, oxo, C₁-C₆alkyloxy, C₁-C₆alkyloxyC₁-C₆alkyl, NR₉R₁₀, C₁-C₆alkylaminoC₁-C₆alkyl substituent wherein the alkyl and aryl groups are optionally substituted as defined in the definition section and R₉ and R₁₀ are defined as above.
The term "arylalkylamino" (e.g. benzylamino, diphenylethylamino and the like) represents one or two "arylalkyl" groups as defined above having the indicated number of carbon atoms attached through an amine bridge. The two "arylalkyl" groups may be taken together with the nitrogen to which they are attached forming a saturated, partially saturated or aromatic cyclic, bicyclic or tricyclic ring system containing 3 to 14 carbon atoms and 0 to 3 additional heteroatoms selected from nitrogen, oxygen or sulfur, the ring system can optionally be substituted with at least one C₁-C₆alkyl, aryl, arylC₁-C₆alkyl, hydroxy, oxo, C₁-C₆alkyloxy, C₁-C₆alkyloxyC₁-C₆alkyl, NR₉R₁₀, C₁-C₆alkylaminoC₁-C₆alkyl substituent wherein the alkyl and aryl groups are optionally substituted as defined in the definition section and R₉ and R₁₀ are defined as above. The term "alkylaminoalkyl" represents an "alkylamino" group attached through an alkyl group as defined above having the indicated number of carbon atoms.
The term "arylalkylaminoalkyl" represents an "arylalkylamino" group attached through an alkyl group as defined above having the indicated number of carbon atoms.
The term "arylalkyl" (e.g. benzyi, phenylethyl) represents an "aryl" group as defined below attached through an alkyl having the indicated number of carbon atoms or substituted alkyl group as defined above.
The term "alkylcarbonyl" (e.g. cyclooctylcarbonyl, pentylcarbonyl, 3-hexenylcarbonyl) represents an "alkyl" group as defined above having the indicated number of carbon atoms attached through a carbonyl group.
The term "arylcarbonyl" (benzoyl) represents an "aryl" group as defined above attached through a carbonyl group.
The term "arylalkylcarbonyl" (e.g. phenylcyclopropylcarbonyl, phenylethylcarbonyl and the like) represents an "arylalkyl" group as defined above having the indicated number of carbon atoms attached through a carbonyl group.
The term "alkylcarbonylalkyl" represents an "alkylcarbonyl" group attached through an "alkyl" group as defined above having the indicated number of carbon atoms.
The term "arylalkylcarbonylalkyl" represents an "arylalkylcarbonyl" group attached through an alkyl group as defined above having the indicated number of carbon atoms.

The term "alkylcarboxy" (e.g. heptylcarboxy, cyclopropylcarboxy, 3-pentenylcarboxy) represents an "alkylcarbonyl" group as defined above wherein the carbonyl is in turn attached through an oxygen bridge.
The term "arylcarboxyalkyl" (e.g. phenylcarboxymethyl) represents an "arylcarbonyl" group defined above wherein the carbonyl is in turn attached through an oxygen bridge to an alkyl chain having the indicated number of carbon atoms.
The term "arylalkylcarboxy" (e.g. benzylcarboxy, phenylcyclopropylcarboxy and the like) represents an "arylalkylcarbonyl" group as defined above wherein the carbonyl is in turn attached through an oxygen bridge.
The term "alkylcarboxyalkyl" represents an "alkylcarboxy" group attached through an "alkyl" group as defined above having the indicated number of carbon atoms.
The term "arylalkylcarboxyalkyl" represents an "arylalkylcarboxy" group attached through an "alkyl" group as defined above having the indicated number of carbon atoms.

The term "alkylcarbonylamino" (e.g. hexylcarbonylamino, cyclopentylcarbonylaminomethyl, methylcarbonylaminophenyl) represents an "alkylcarbonyl" group as defined above wherein the carbonyl is in turn attached through the nitrogen atom of an amino group. The nitrogen atom may itself be substituted with an alkyl or aryl group.
The term "arylalkylcarbonylamino" (e.g. benzylcarbonylamino and the like) represents an "arylalkylcarbonyl" group as defined above wherein the carbonyl is in turn attached through the nitrogen atom of an amino group. The nitrogen atom may itself be substituted with an alkyl or aryl group.
The term "alkylcarbonylaminoalkyl" represents an "alkylcarbonylamino" group attached through an "alkyl" group as defined above having the indicated number of carbon atoms. The nitrogen atom may itself be substituted with an alkyl or aryl group. The term "arylalkylcarbonylaminoalkyl" represents an "arylalkylcarbonylamino" group attached through an "alkyl" group as defined above having the indicated number of carbon atoms. The nitrogen atom may itself be substituted with an alkyl or aryl group.

The term "alkylcarbonylaminoalkylcarbonyl" represents an alkylcarbonylaminoalkyl group attached through a carbonyl group. The nitrogen atom may be further substituted with an "alkyl" or "aryl" group.
The term "aryl" represents an unsubstituted, mono-, di- or trisubstituted monocyclic, polycyclic, biaryl and heterocyclic aromatic groups covalently attached at any ring position capable of forming a stable covalent bond, certain preferred points of attachment being apparent to those skilled in the art (e.g., 3-indolyl, 4-imidazolyl). The aryl substituents are independently selected from the group consisting of halo, nitro, cyano, trihalomethyl, C₁-C₆alkyl, aryl, arylC₁-C₆alkyl, hydroxy, COR₅, C₁-C₆alkyloxy, C₁-C₆alkyloxyC₁-C₆alkyl, aryloxy, arylC₁-C₆alkyloxy, arylC₁-C₆alkyloxyC₁-C₆alkyl, thio, C₁-C₆alkylthio, C₁-C₆alkylthioC₁-C₆alkyl, arylthio, arylC₁-C₆alkylthio, arylC₁-C₆alkylthioC₁-C₆alkyl, NR₈R₉, C₁-C₆-alkylamino, C₁-C₆alkylaminoC₁-C₆alkyl, arylamino, arylC₁-C₆alkylamino, arylC₁-C₆alkyl-aminoC₁-C₆alkyl, di(arylC₁-C₆alkyl)aminoC₁-C₆alkyl, C₁-C₆alkylcarbonyl, C₁-C₆alkylcarbonylC₁-C₆alkyl, arylC₁-C₆alkylcarbonyl, arylC₁-C₆alkyl-carbonylC₁-C₆alkyl, C₁-C₆alkylcarboxy, C₁-C₆alkylcarboxy-C₁-C₆alkyl, arylC₁-C₆alkylcarboxy, arylC₁-C₆alkylcarboxyC₁-C₆alkyl, carboxyC₁-C₆alkyloxy, C₁-C₆alkylcarbonylamino, C₁-C₆alkylcarbonylaminoC₁-C₆alkyl, -carbonylNR₇C₁-C₆alkylCOR₁₁, arylC₁-C₆alkylcarbonylamino, arylC₁-C₆alkylcarbonylaminoC₁-C₆alkyl, -CONR₈R₉, or -C₁-C₆alkylCONR₈R₉; wherein R₇, R₈, R₉, and R₁₁ are defined as above.

The definition of aryl includes phenyl, biphenyl, indenyl, fluorenyl, naphthyl (1-naphthyl, 2-naphthyl), pyrrolyl (2-pyrrolyl), pyrazolyl (3-pyrazolyl), imidazolyl (1-imidazolyl, 2-imidazolyl, 4-imidazolyl, 5-imidazolyl), triazolyl (1,2,3-triazol-1-yl, 1,2,3-triazol-2-yl 1,2,3-triazol-4-yl, 1,2,4-triazol-3-yl), oxazolyl (2-oxazolyl, 4-oxazolyl, 5-oxazolyl), isoxazolyl (3-isoxazolyl, 4-isoxazolyl, 5-isoxazolyl), thiazolyl (2-thiazolyl, 4-thiazolyl, 5-thiazolyl), thiophenyl (2-thiophenyl, 3-thiophenyl, 4-thiophenyl, 5-thiophenyl), furanyl (2-furanyl, 3-furanyl, 4-furanyl, 5-furanyl), pyridyl (2-pyridyl, 3-pyridyl, 4-pyridyl, 5-pyridyl), 5-tetrazolyl, pyrimidinyl (2-pyrimidinyl, 4-pyrimidinyl, 5-pyrimidinyl, 6-pyrimidinyl), pyrazinyl, pyridazinyl (3-pyridazinyl, 4-pyridazinyl, 5-pyridazinyl), quinolyl (2-quinolyl, 3-quinolyl, 4-quinolyl, 5-quinolyl, 6-quinolyl, 7-quinolyl, 8-quinolyl), isoquinolyl (1-isoquinolyl, 3-isoquinolyl, 4-isoquinolyl, 5-isoquinolyl, 6-isoquinolyl, 7-isoquinolyl, 8-isoquinolyl), benzo[b]furanyl (2-benzo[b]furanyl, 3-benzo[b]furanyl, 4-benzo[b]furanyl, 5-benzo[b]furanyl, 6-benzo[b]furanyl, 7-benzo[b]furanyl), 2,3-dihydro-benzo[b]furanyl (2-(2,3-dihydro-benzo[b]furanyl), 3-(2,3-dihydro-benzo[b]furanyl), 4-(2,3-dihydro-benzo[b]furanyl), 5-(2,3-dihydro-benzo-[b]furanyl), 6-(2,3-dihydro-benzo-[b]furanyl), 7-(2,3-dihydro-benzo[b]furanyl)), benzo[b]thiophenyl (2-benzo[b]thiophenyl, 3-benzo[b]thiophenyl, 4-benzo[b]thiophenyl, 5-benzo[b]thiophenyl, 6-benzo[b]thiophenyl, 7-benzo[b]thiophenyl), 2,3-dihydro-benzo[b]-thiophenyl (2-(2,3-dihydro-benzo[b]thiophenyl), 3-(2,3-dihydro-benzo[b]-thiophenyl), 4-(2,3-dihydro-benzo[b]thiophenyl), 5-(2,3-dihydro-benzo[b]-thiophenyl), 6-(2,3-dihydro-benzo[b]thiophenyl), 7-(2,3-dihydro-benzo[b]-thiophenyl)), 4,5,6,7-tetrahydro-benzo[b]thiophenyl (2-(4,5,6,7-tetrahydro-benzo-[b]thiophenyl), 3-(4,5,6,7-tetrahydro-benzo-[b]thiophenyl), 4-(4,5,6,7-tetrahydro-benzo[b]thiophenyl), 5-(4,5,6,7-tetrahydro-benzo-[b]thiophenyl), 6-(4,5,6,7-tetrahydro-benzo-[b]thiophenyl), 7-(4,5,6,7-tetrahydro-benzo[b]thiophenyl)), 4,5,6,7-tetrahydro-thieno[2,3-c]pyridyl (4-(4,5,6,7-tetrahydro-thieno[2,3-c]pyridyl), 5-4,5,6,7-tetrahydro-thieno[2,3-c]pyridyl), 6-(4,5,6,7-tetrahydro-thieno[2,3-c]pyridyl), 7-(4,5,6,7-tetrahydro-thieno[2,3-c]pyridyl)), indolyl (1-indolyl, 2-indolyl, 3-indolyl, 4-indolyl, 5-indolyl, 6-indolyl, 7-indolyl), isoindolyl (1-isoindolyl, 2-isoindolyl, 3-isoindolyl, 4-isoindolyl, 5-isoindolyl, 6-isoindolyl, 7-isoindolyl), 1,3-dihydro-isoindolyl (1-(1,3-dihydro-isoindolyl), 2-(1,3-dihydro-isoindolyl), 3-(1,3-dihydro-isoindolyl), 4-(1,3-dihydro-isoindolyl), 5-(1,3-dihydro-isoindolyl), 6-(1,3-dihydro-isoindolyl), 7-(1,3-dihydro-isoindolyl)), indazole (1-indazolyl, 3-indazolyl, 4-indazolyl, 5-indazolyl, 6-indazolyl, 7-indazolyl), benzimidazolyl (1-benzimidazolyl, 2-benzimidazolyl, 4-benzimidazolyl, 5-benzimidazolyl, 6-benzimidazolyl, 7-benzimidazolyl, 8-benzimidazolyl), benzoxazolyl (1-benz-oxazolyl, 2-benzoxazolyl), benzothiazolyl (1-benzothiazolyl, 2-benzo-thiazolyl, 4-benzothiazolyl, 5-benzothiazolyl, 6-benzothiazolyl, 7-benzothiazolyl), carbazolyl (1-carbazolyl, 2-carbazolyl, 3-carbazolyl, 4-carbazolyl), 5H-dibenz[b,f]azepine (5H-dibenz[b,f]azepin-1-yl, 5H-dibenz-[b,f]azepine-2-yl, 5H-dibenz[b,f]azepine-3-yl, 5H-dibenz-[b,f]azepine-4-yl, 5H-dibenz[b,f]-azepine-5-yl), 10,11-dihydro-5H-dibenz[b,f]azepine (10,11-dihydro-5H-dibenz[b,f]azepine-1-yl, 10,11-dihydro-5H-dibenz[b,f]azepine-2-yl, 10,11-dihydro-5H-dibenz[b,f]azepine-3-yl, 10,11-dihydro-5H-dibenz-[b,f]azepine-4-yl, 10,11-dihydro-5H-dibenz[b,f]azepine-5-yl), piperidinyl (2-piperidinyl, 3-piperidinyl, 4-piperidinyl), pyrrolidinyl (1-pyrrolidinyl, 2-pyrrolidinyl, 3-pyrrolidinyl), phenylpyridyl (2-phenyl-pyridyl, 3-phenyl-pyridyl, 4-phenylpyridyl), phenylpyrimidinyl (2-phenylpyrimidinyl, 4-phenyl-pyrimidinyl, 5-phenylpyrimidinyl, 6-phenylpyrimidinyl), phenylpyrazinyl, phenylpyridazinyl (3-phenylpyridazinyl, 4-phenylpyridazinyl, 5-phenyl-pyridazinyl).

The term "arylcarbonyl" (e.g. 2-thiophenylcarbonyl, 3-methoxy-anthrylcarbonyl, oxa-zolylcarbonyl) represents an "aryl" group as defined above attached through a carbonyl group.
The term "arylalkylcarbonyl" (e.g. (2,3-dimethoxyphenyl)propylcarbonyl, (2-chloronaphthyl)pentenylcarbonyl, imidazolylcyclopentylcarbonyl) represents an "arylalkyl" group as defined above wherein the "alkyl" group is in turn attached through a carbonyl.
The compounds of the present invention have asymmetric centers and may occur as racemates, racemic mixtures, and as individual enantiomers or diastereoisomers, with all isomeric forms being included in the present invention as well as mixtures thereof.

Pharmaceutically acceptable salts of the compounds of formula 1, where a basic or acidic group is present in the structure, are also included within the scope of this invention. When an acidic substituent is present, such as -COOH, 5-tetrazolyl or -P(O)(OH)₂, there can be formed the ammonium, morpholinium, sodium, potassium, barium, calcium salt, and the like, for use as the dosage form. When a basic group is present, such as amino or a basic heteroaryl radical, such as pyridyl, an acidic salt, such as hydrochloride, hydrobromide, phosphate, sulfate, trifluoroacetate, trichloroacetate, acetate, oxalate, maleate, pyruvate, malonate, succinate, citrate, tartarate, fumarate, mandelate, benzoate, cinnamate, methanesulfonate, ethane sulfonate, picrate and the like, and include acids related to the pharmaceutically acceptable salts listed in Journal of Pharmaceutical Science, 66, 2 (1977) and incorporated herein by reference, can be used as the dosage form.
Also, in the case of the -COOH or -P(O)(OH)₂ being present, pharmaceutically acceptable esters can be employed, e.g., methyl, tert-butyl, pivaloyloxymethyl, and the like, and those esters known in the art for modifying solubility or hydrolysis characteristics for use as sustained release or prodrug formulations.
In addition, some of the compounds of the instant invention may form solvates with water or common organic solvents. Such solvates are encompassed within the scope of the invention.
The term "therapeutically effective amount" shall mean that amount of drug or pharmaceutical agent that will elicit the biological or medical response of a tissue, system, animal, or human that is being sought by a researcher, veterinarian, medical doctor or other.

### PREFERRED EMBODIMENTS OF THE INVENTION

Compounds of Formula 1a are preferred compounds of the invention 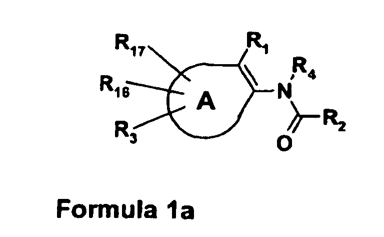 wherein
A is together with the double bond in Formula 1a 4,5,6,7-tetrahydro-thieno[2,3-*b*]pyridyl, 4,5,6,7-tetrahydro-thieno[2,3-*c*]pyridyl, 4,5,6,7-tetrahydro-thieno[3,2-*c*]pyridyl, 4,5,6,7-tetrahydro-thieno[3,2-*b*]pyridyl, or 4,5,6,7-tetrahydro-4,7-ethanothieno[2,3-b]pyridyl;
R₁ is COR₅, OR₆, CF₃, nitro, SO₃H, SO₂NR₇R₈, PO(OH)₂, CH₂PO(OH)₂, CHFPO(OH)₂, CF₂PO(OH)₂, C(=NH)NH₂, NR₇R₈ or selected from the following 5-membered heterocycles: 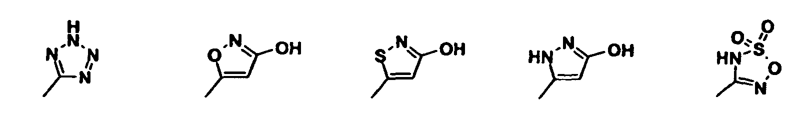 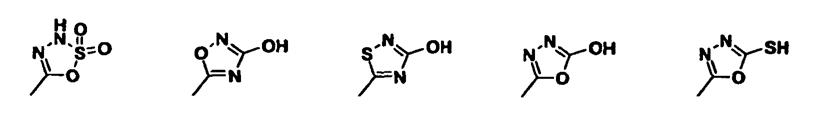 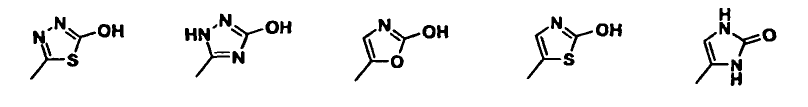 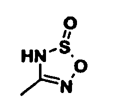 or R₁ is 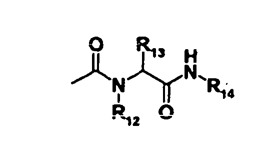 wherein R₁₂, R₁₃, and R₁₄ are independently hydrogen, C₁-C₆alkyl, aryl, arylC₁-C₆alkyl and the alkyl and aryl groups are optionally substituted;
R₂ is COR₅, OR₆, CF₃, nitro, cyano, SO₃H, SO₂NR₇R₈, PO(OH)₂, CH₂PO(OH)₂, CHFPO(OH)₂, CF₂PO(OH)₂, C(=NH)NH₂, NR₇R₈, or selected from the following 5-membered heterocycles: 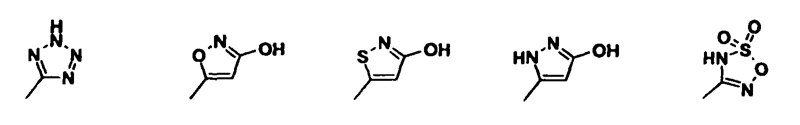 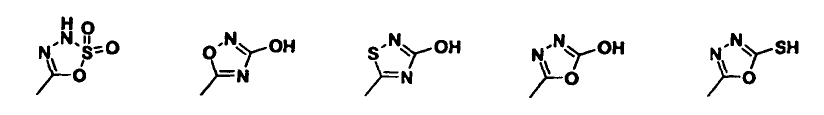 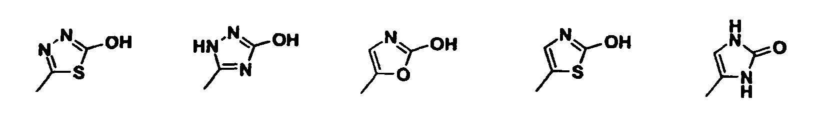 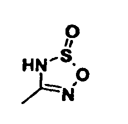 R₃, R₁₆ and R₁₇ are independently hydrogen, halo, nitro, cyano, trihalomethyl, C₁-C₆alkyl, aryl, arylC₁-C₆-alkyl, hydroxy, carboxy, carboxyC₁-C₆alkyl, C₁-C₆alkyloxycarbonyl, aryloxycarbonyl, arylC₁-C₆alkyloxycarbonyl, C₁-C₆alkyloxy, C₁-C₆alkyloxyC₁-C₆alkyl, aryloxy, arylC₁-C₆alkyloxy, arylC₁-C₆alkyl-oxyC₁-C₆alkyl, thio, C₁-C₆alkylthio, C₁-C₆alkylthioC₁-C₆alkyl, arylthio, arylC₁-C₆alkylthio, arylC₁-C₆alkylthioC₁-C₆alkyl, NR₇R₈, C₁-C₆alkyl-aminoC₁-C₆alkyl, arylC₁-C₆alkylaminoC₁-C₆alkyl, di(arylC₁-C₆alkyl)-aminoC₁-C₆alkyl, C₁-C₆alkylcarbonyl, C₁-C₆alkylcarbonylC₁-C₆alkyl, arylC₁-C₆alkylcarbonyl, arylC₁-C₆alkylcarbonylC₁-C₆alkyl, C₁-C₆alkyl-carboxy, C₁-C₆alkylcarboxyC₁-C₆-alkyl, arylcarboxy, arylC₁-C₆alkyl-carboxy, arylC₁-C₆alkylcarboxyC₁-C₆alkyl, C₁-C₆alkylcarbonylamino, C₁-C₆alkylcarbonyl-aminoC₁-C₆alkyl, -carbonylNR₇C₁-C₆alkylCOR₁₁, arylC₁-C₆alkyl-carbonylamino, arylC₁-C₆alkylcarbonylaminoC₁-C₆alkyl, CONR₇R₈, or C₁-C₆alkylCONR₇R₈ wherein the alkyl and aryl groups are optionally substituted and R₁₁ is NR₇R₈, or C₁-C₆alkylNR₇R₈; or R₃ is 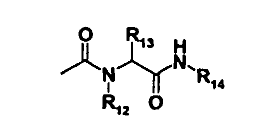 wherein R₁₂, R₁₃, and R₁₄ are independently hydrogen, C₁-C₆alkyl, aryl, arylC₁-C₆alkyl and the alkyl and aryl groups are optionally substituted;
R₄ is hydrogen, hydroxy, C₁-C₆alkyl, aryl, arylC₁-C₆alkyl, NR₇R₈, C₁-C₆alkyloxy; wherein the alkyl and aryl groups are optionally substituted;
R₅ is hydroxy, C₁-C₆alkyl, aryl, arylC₁-C₆alkyl, CF₃; wherein the alkyl and aryl groups are optionally substituted;
R₆ is hydrogen, C₁-C₆alkyl, aryl, arylC₁-C₆alkyl; wherein the alkyl and aryl groups are optionally substituted;
R₇ and R₈ are independently selected from hydrogen, C₁-C₆alkyl, aryl, arylC₁-C₆alkyl, C₁-C₆alkyl-carbonyl, arylcarbonyl, arylC₁-C₆alkyl-carbonyl, C₁-C₆alkyl-carboxy or arylC₁-C₆alkylcarboxy wherein the alkyl and aryl groups are optionally substituted; or R₇ and R₈ are taken together with the nitrogen to which they are attached forming a cyclic or bicyclic system containing 3 to 11 carbon atoms and 0 to 2 additional heteroatoms selected from nitrogen, oxygen or sulfur, the ring system can optionally be substituted with at least one C₁-C₆alkyl, aryl, arylC₁-C₆alkyl, hydroxy, C₁-C₆alkyloxy, arylC₁-C₆alkyloxy, C₁-C₆alkyloxyC₁-C₆alkyl, NR₉R₁₀ or C₁-C₆alkylamino-C₁-C₆alkyl, wherein R₉ and R₁₀ are independently selected from hydrogen, C₁-C₆alkyl, aryl, arylC₁-C₆alkyl, C₁-C₆alkylcarbonyl, arylcarbonyl, arylC₁-C₆alkylcarbonyl, C₁-C₆alkylcarboxy or arylC₁-C₆alkylcarboxy; wherein the alkyl and aryl groups are optionally substituted; or
R₇ and R₈ are independently a saturated or partial saturated cyclic 5, 6 or 7 membered amine or lactam.

Further, preferred compounds of the invention are compounds of formula la wherein R₁₆ and R₁₇ are hydrogen.

Particular preferred compounds of the invention are those compounds of formula I wherein R₁ is 5-tetrazolyl, i.e. 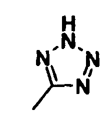 or COR₅ and R₂ is COR₅.
In particular, preferred compounds are those wherein R₅ is OH and R₄ is hydrogen.

The following compounds are preferred:
6-Benzoyl-2-(oxalyl-amino)-4,5,6,7-tetrahydro-thieno[2,3-*c*]pyridine-3-carboxylic acid;
6-Benzyl-2-(oxalyl-amino)-4,5,6,7-tetrahydro-thieno[2,3-*c*]pyridine-3-carboxylic acid;
6-Methyl-2-(oxalyl-amino)-4,5,6,7-tetrahydro-thieno[2,3-*c*]pyridine-3-carboxylic acid;
2-(Oxalyl-amino)-6-phenethyl-4,5,6,7-tetrahydro-thieno[2,3-*c*]pyridine-3-carboxylic acid;
5-Benzoyl-2-(oxalyl-amino)-4,5,6,7-tetrahydro-thieno[3,2-*c*]pyridine-3-carboxylic acid;
5-Benzyl-2-(oxalyl-amino)-4,5,6,7-tetrahydro-thieno[3,2-*c*]pyridine-3-carboxylic acid;
5-Methyl-2-(oxalyl-amino)-4,5,6,7-tetrahydro-thieno[3,2-*c*]pyridine-3-carboxylic acid;
2-(Oxalyl-amino)-5-phenethyl-4,5,6,7-tetrahydro-thieno[3,2-*c*]pyridine-3-carboxylic acid;
2-(Oxalyl-amino)-4,5,6,7-tetrahydro-4,7-ethano-thieno[2,3-b]pyridine-3-carboxylic acid;
2-(Oxalyl-amino)-6-pyridin-2-ylmethyl-4,5,6,7-tetrahydro-thieno[2,3-c]pyridine-3-carboxylic acid;
2-(Oxalyl-amino)-4,5,6,7-tetrahydro-thieno[2,3-c]pyridine-3-carboxylic acid;
6-(3-Methoxy-benzyl)-2-(oxalyl-amino)-4,5,6,7-tetrahydro-thieno[2,3-c]pyridine-3-carboxylic acid;
2-(Oxalyl-amino)-6-pyridin-3-ylmethyl-4,5,6,7-tetrahydro-thieno[2,3-c]pyridine-3-carboxylic acid;
2-(Oxalyl-amino)-6-pyridin-4-ylmethyl-4,5,6,7-tetrahydro-thieno[2,3-c]pyridine-3-carboxylic acid;
2-(Oxalyl-amino)-6-quinolin-2-ylmethyl-4,5,6,7-tetrahydro-thieno[2,3-c]pyridine-3-carboxylic acid;
2-(Oxalyl-amino)-4,7-dihydro-5H-thieno[2,3-c]pyridine-3,6-dicarboxylic acid 6-benzyl ester;
6-Acetyl-2-(oxalyl-amino)-4,5,6,7-tetrahydro-thieno[2,3-c]pyridine-3-carboxylic acid;
2-(Oxalyl-amino)-6-phenylcarbamoylmethyl-4,5,6,7-tetrahydro-thieno[2,3-c]pyridine-3-carboxylic acid;

### PHARMACOLOGICAL METHODS

The compounds are evaluated for biological activity with a truncated form of PTP1B (corresponding to the first 321 amino acids), which was expressed in E. coli and purified to apparent homogeneity using published procedures well-known to those skilled in the art. The enzyme reactions are carried out using standard conditions essentially as described by Burke et al. (*Biochemistry* **35**; 15989-15996 (1996)). The assay conditions are as follows. Appropriate concentrations of the compounds of the invention are added to the reaction mixtures containing different concentrations of the substrate, *p*-nitrophenyl phosphate (range: 0.16 to 10 mM - final assay concentration). The buffer used was 100 mM sodium acetate pH 5.5, 50 mM sodium chloride, 0.1 % (w/v) bovine serum albumin and 5 mM dithiothreitol (total volume 100 ml). The reaction was started by addition of the enzyme and carried out in microtiter plates at 25° C for 60 minutes. The reactions are stopped by addition of NaOH. The enzyme activity was determined by measurement of the absorbance at 405 nm with appropriate corrections for absorbance at 405 nm of the compounds and *p*-nitrophenyl phosphate. The data are analyzed using nonlinear regression fit to classical Michaelis Menten enzyme kinetic models. Inhibition is expressed as Kᵢ values in µM. The results of representative experiments are shown in Table 1

**Table 1**

| Inhibition of classical PTP1B by compounds of the invention | |
|---|---|
| **Example no.** | **PTP1B** |
| | **K**_{**i**} **values (µM)** |
| 1 | 51 |
| 4 | 3 |

Further, the compounds are evaluated for biological activity as regards their effect as inhibitors of PTPα in essentially the same way as described for inhibition of PTP1B. Derived from their activity as evaluated above the compounds of the invention may be useful in the treatment of diseases selected from the group consisting of type I diabetes, type II diabetes, impaired glucose tolerance, insulin resistance and obesity. Furthermore, derived from their activity as evaluated above, the compounds of the invention may be useful in the treatment of diseases selected from the group consisting of immune dysfunctions including autoimmunity, diseases with dysfunctions of the coagulation system, allergic diseases including asthma, osteoporosis, proliferative disorders including cancer and psoriasis, diseases with decreased or increased synthesis or effects of growth hormone, diseases with decreased or increased synthesis of hormones or cytokines that regulate the release of/or response to growth hormone, diseases of the brain including Alzheimer's disease and schizophrenia, and infectious diseases.

### THE SYNTHESIS OF THE COMPOUNDS

In accordance with one aspect of the invention, the compounds of the invention are prepared as illustrated in the following reaction scheme:

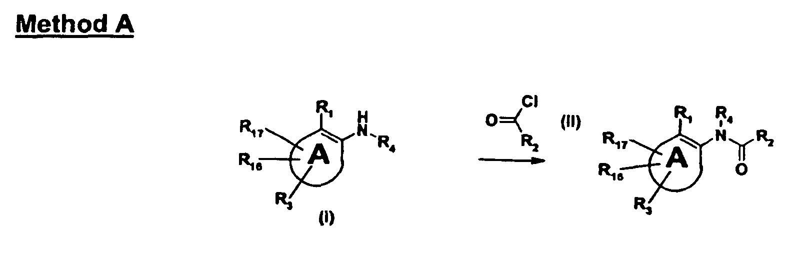

By allowing an amino substituted aryl or heteroaryl (I) to react with an acid chloride of formula (II), wherein A, R₁, R₂, R₃, R₄, R₁₆ and R₁₇ are defined as above.

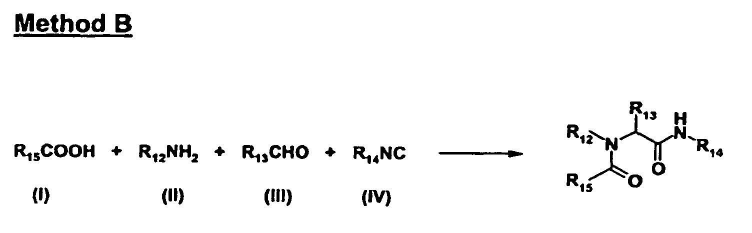

By allowing a carboxylic acid (I), a primary amine (II) and an aldehyde (III) to react with a isocyanide (IV) wherein R₁₂, R₁₃, R₁₄, and R₁₅ are independently selected from the group consisting of hydrogen, C₁-C₆alkyl, aryl, arylC₁-C₆alkyl as defined above and the alkyl and aryl groups are optionally substituted as defined above; or R₁₂, R₁₃, R₁₄, and R₁₅ are independently selected from 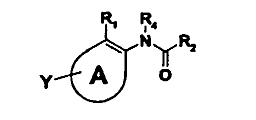 wherein Y indicates attachment point for R₁₂, R₁₃, R₁₄, and R₁₅ and A, R₁ R₂ and R₄ are defined as above.
In a preferred method, the above described four component Ugi reaction can be carried out by attaching any one of the components to a solid support. Hence, the synthesis can be accomplished in a combinatorial chemistry fashion.

General procedure for the Preparation of Acetoxymethyl Esters (C.Schultz et al, The Journal of Biological Chemistry, **1993,** 268, 6316-6322.): A carboxylic acid (1 equivalent) was suspended in dry acetonitrile (2 ml per 0.1 mmol). Diisopropyl amine (3.0 equivalents) was added followed by bromomethyl acetate (1.5 equivalents). The mixture was stirred under nitrogen overnight at room temperature. Acetonitrile was removed under reduced pressure to yield an oil which was diluted in ethylacetate and washed water (3 x). The organic layer was dried over anhydrous magnesium sulfate. Filtration followed by solvent removal under reduced pressure afforded a crude oil. The product was purified by column chromatography on silica gel, using an appropriate solvent system.

The present invention also has the objective of providing suitable topical, oral, and parenteral pharmaceutical formulations for use in the novel methods of treatment of the present invention. The compounds of the present invention may be administered orally as tablets, aqueous or oily suspensions, lozenges, troches, powders, granules, emulsions, capsules, syrups or elixirs. The composition for oral use may contain one or more agents selected from the group of sweetening agents, flavouring agents, colouring agents and preserving agents in order to produce pharmaceutically elegant and palatable preparations. The tablets contain the acting ingredient in admixture with non-toxic pharmaceutically acceptable excipients which are suitable for the manufacture of tablets. These excipients may be, for example, (1) inert diluents, such as calcium carbonate, lactose, calcium phosphate or sodium phosphate; (2) granulating and disintegrating agents, such as corn starch or alginic acid; (3) binding agents, such as starch, gelatin or acacia; and (4) lubricating agents, such as magnesium stearate, stearic acid or talc. These tablets may be uncoated or coated by known techniques to delay disintegration and absorption in the gastrointestinal tract and thereby provide a sustained action over a longer period. For example, a time delay material such as glyceryl monostearate or glyceryl distearate may be employed. Coating may also be performed using techniques described in the U.S. Patent Nos. 4,256,108; 4,160,452; and 4,265,874 to form osmotic therapeutic tablets for control release.
Formulations for oral use may be in the form of hard gelatin capsules wherein the active ingredient is mixed with an inert solid diluent, for example, calcium carbonate, calcium phosphate or kaolin. They may also be in the form of soft gelatin capsules wherein the active ingredient is mixed with water or an oil medium, such as peanut oil, liquid paraffin or olive oil.
Aqueous suspensions normally contain the active materials in admixture with excipients suitable for the manufacture of aqueous suspension. Such expicients may be (1) suspending agent such as sodium carboxymethyl cellulose, methyl cellulose, hydroxypropylmethyl-cellulose, sodium alginate, polyvinylpyrrolidone, gum tragacanth and gum acacia; (2) dispersing or wetting agents which may be (a) naturally occurring phosphatide such as lecithin; (b) a condensation product of an alkylene oxide with a fatty acid, for example, polyoxyethylene stearate; (c) a condensation product of ethylene oxide with a long chain aliphatic alcohol, for example, heptadecaethylenoxycetanol; (d) a condensation product of ethylene oxide with a partial ester derived from a fatty acid and hexitol such as polyoxyethylene sorbitol monooleate, or (e) a condensation product of ethylene oxide with a partial ester derived from fatty acids and hexitol anhydrides, for example polyoxyethylene sorbitan monooleate.
The pharmaceutical compositions may be in the form of a sterile injectable aqueous or oleagenous suspension. This suspension may be formulated according to known methods using those suitable dispersing or wetting agents and suspending agents which have been mentioned above. The sterile injectable preparation may also a sterile injectable solution or suspension in a non-toxic parenterally-acceptable diluent or solvent, for example, as a solution in 1,3-butanediol. Among the acceptable vehicles and solvents that may be employed are water, Ringer's solution, and isotonic sodium chloride solution. In addition, sterile, fixed oils are conventionally employed as a solvent or suspending medium. For this purpose, any bland fixed oil may be employed including synthetic mono- or diglycerides. In addition, fatty acids such as oleic acid find use in the preparation of injectables.
The Compounds of the invention may also be administered in the form of suppositories for rectal administration. These compositions can be prepared by mixing the drug with a suitable non-irritating excipient which is solid at ordinary temperature but liquid at the rectal temperature and will therefore melt in the rectum to release the drug. Such materials are cocoa butter and polyethylene glycols.
The compounds of the present invention may also be administered in the form of liposome delivery systems, such as small unilamellar vesicles, large unilamellar vesicles, and multilamellar vesicles. Liposomes can be formed from a variety of phospholipids, such as cholesterol, stearylamine, or phosphatidyl-cholines.
For topical use, creams, ointments, jellies, solutions or suspensions, etc., containing the compounds of Formula 1 are employed.
Dosage levels of the compounds of the present invention are of the order of about 0.5 mg to about 100 mg per kilogram body weight, with a preferred dosage range between about 20 mg to about 50 mg per kilogram body weight per day (from about 25 mg to about 5 g's per patient per day). The amount of active ingredient that may be combined with the carrier materials to produce a single dosage will vary depending upon the host treated and the particular mode of administration. For example, a formulation intended for oral administration to humans may contain 5 mg to 1 g of an active compound with an appropriate and convenient amount of carrier material which may vary from about 5 to about 95 percent of the total composition. Dosage unit forms will generally contain between from about 5 mg to about 500 mg of active ingredient.
It will be understood, however, that the specific dose level for any particular patient will depend upon a variety of factors including the activity of the specific compound employed, the age, body weight, general health, gender, diet, time of administration, route of administration, rate of excretion, drug combination and the severity of the particular disease undergoing therapy. The dosage needs to be individualized by the clinician.

### EXAMPLES

The process for preparing compounds of Formula 1 and preparations containing them is further illustrated in the following examples, which, however, are not to be construed as limiting.

Hereinafter, TLC is thin layer chromatography, CDCl₃ is deuterio chloroform, CD₃OD is tetradeuterio methanol and DMSO-d₆ is hexadeuterio dimethylsulfoxide. The structures of the compounds are confirmed by either elemental analysis or NMR, where peaks assigned to characteristic protons in the title compounds are presented where appropriate. ¹H NMR shifts (δ_{H}) are given in parts per million (ppm) down field from tetramethylsilane as internal reference standard. M.p.: is melting point and is given in °C and is not corrected. Column chromatography was carried out using the technique described by W.C. Still *et al., J. Org. Chem. 43*: 2923 (1978) on Merck silica gel 60 (Art. 9385). HPLC analyses are performed using 5µm C18 4 x 250 mm column eluted with various mixtures of water and acetonitrile, flow = 1 ml/min, as described in the experimental section.
Wang-resin is polystyrene with a 4-hydroxymethylphenol ether linker.
Compounds used as starting material are either known compounds or compounds which can readily be prepared by methods known per se.

### EXAMPLE 1

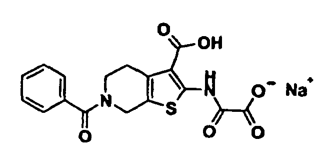

### 6-Benzoyl-2-(oxalyl-amino)-4,5,6,7-tetrahydro-thieno[2,3-c]pyridine-3-carboxylic acid, mono sodium salt;

A mixture of N-benzoyl-4-piperidone (20.0 g, 0.1 mol), ethyl cyanoacetate (10.9 ml, 0.1 mol), ammonium acetate (2.0 g) and acetic acid (6 ml) in benzene (100 ml) was heated at reflux temperature in a 3-nacked reaction flask equipped with a Dean-Stark water trap for 1 h. The cooled reaction mixture was diluted with ethyl acetate (100 ml) washed with water (3 x 100 ml), saturated aqueous sodium chloride (80 ml), dried (MgSO₄) filtered and evaporated in vacuo affording quantitative yield of (1-benzoyl-piperidin-4-ylidene)-cyano-acetic acid ethyl ester as a slowly crystallising oil.

A mixture of the above benzoyl-piperidin-4-ylidene (10.0 g, 0.034 mol), sulphur (1.13 g, 0.035 mol) and morpholin (6.5 ml) in ethanol (35 ml) was heated at 50 °C for 2 h and stirred at room temperature over night. The precipitate was filtered off and washed with 96 % ethanol (3 x 50 ml), diethyl ether (3 x 50 ml) and dried in vacuo which afforded 9.27 g (84 %) of 2-amino-6-benzoyl-4,5,6,7-tetrahydro-thieno[2,3-c]pyridine-3-carboxylic acid ethyl ester as a solid.

To a stirred solution of the above 4,5,6,7-tetrahydro-thieno[2,3-c]pyridine-3-carboxylic acid ethyl ester (5.0 g, 0.015 mol), triethylamine (4.21 ml, 0.03 mol) in dry tetrahydrofuran (30 ml) at 0 °C was added dropwise a solution of ethyl oxalyl chloride (1.9 ml, 0.017 mol) in dry tetrahydrofuran (20 ml). The resulting reaction mixture was stirred at room temperature for 18 h, poured into ice water (300 ml) and extracted with ethyl acetate (3 x 100 ml). The combined organic extracts were washed with saturated aqueous sodium chloride (100 ml), dried (MgSO₄) filtered and evaporated in vacuo affording 4.2 g (84 %) of 6-benzoyl-2-(ethoxyoxalyl-amino)-4,5,6,7-tetrahydro-thieno[2,3-c]pyridine-3-carboxylic acid ethyl ester as a crystallising oil.

To a solution of the above thieno[2,3-c]pyridine-3-carboxylic acid ethyl ester (4.2 g, 9.76 mmol) in ethanol (100 ml) was added a solution of sodium hydroxide (0.9 g, 21.46 mmol) in water (100 ml). The resulting reaction mixture was stirred at room temperature for 18 h. The volatiles were evaporated in vacuo and the residue dissolved in water (100 ml) and washed with ethyl acetate (2 x 100 ml). To the aqueous phase was added concentrated hydrochloric acid to pH = 1 and the precipitate was filtered off and washed with water (2 x 50 ml), diethyl ether (2 x 30 ml) and dried in vacuo at 50 °C affording 2.9 g (79 %) of the title compound as a solid.

M.p.: Amorph:
Calculated for C₁₇H₁₃N₂O₆S₁Na₁, 1 x H₂O ;
C, 49.28 %; H, 3.65 %; N, 6.76%. Found:
C, 49.31 %; H, 3.86 %; N, 6.53%.

By a similar procedure as described in Example 1 the following compounds have been prepared.

### EXAMPLE 2

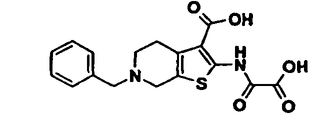

### 6-Benzyl-2-(oxalyl-amino)-4,5,6,7-tetrahydro-thieno[2,3-c]pyridine-3-carboxylic acid;

Calculated for C₁₇H₁₆N₂O₅S, 1.75 H₂O;
C, 52.10 %; H, 5.01 %; N, 7.15 %. Found:
C, 52.11 %; H, 4.81 %; N, 7.01 %.

### EXAMPLE 3

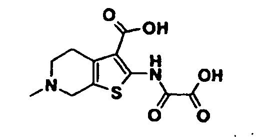

### 6-Methyl-2-(oxalyl-amino)-4,5,6,7-tetrahydro-thieno[2,3-c]pyridine-3-carboxylic acid:

M.p.: > 250 °C
Calculated for C₁₁H₁₂N₂O₅S, 0.6 H₂O;
C, 44.77 %; H, 4.51 %; N, 9.49 %. Found:
C, 44.54 %; H, 4.17 %; N, 9.21 %.

### EXAMPLE 4

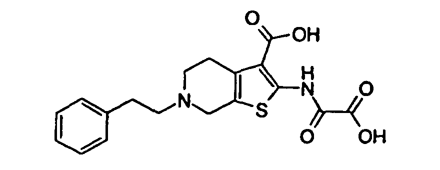

### 2-(Oxalyl-amino)-6-phenethyl-4,5,6,7-tetrahydro-thieno[2,3-c]pyridine-3-carboxylic acid;

Calculated for C₁₈H₁₈N₂O₅S, 1 x H₂O;
C, 55.09 %; H, 5.14 %; N, 7.14 %. Found:
C, 55.47 %; H, 5.04 %; N, 7.07 %.

### EXAMPLE 5

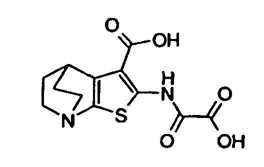

### 2-(Oxalyl-amino)-4,5,6,7-tetrahydro-4,7-ethano-thieno[2,3-b]pyridine-3-carboxylic acid;

Calculated for C₁₂H₁₂N₂O₅S, 0.75 x H₂O;
C, 46.52 %; H, 4.39 %; N, 9.04 %. Found:
C, 46.48 %; H, 4.79 %; N, 8.87 %.

### EXAMPLE 6

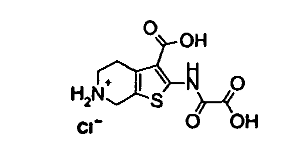

### 2-(Oxalyl-amino)-4,5,6,7-tetrahydro-thieno[2,3-c]pyridine-3-carboxylic acid, hydrochloride;

4-Oxo-1-piperidine carboxylic acid *tert*-butyl ester was used as starting material. The Boc-group was removed using 25% trifluoroacetic acid in dichloromethane.
M.p.: > 250 °C
Calculated for C₁₀H₁₀N₂O₅S, 1 HCl, 0.5 x H₂O;
C, 38.35 %; H, 4.34 %; N, 8.64 %. Found:
C, 38.04 %; H, 3.83 %; N, 8.87 %.

### EXAMPLE 7

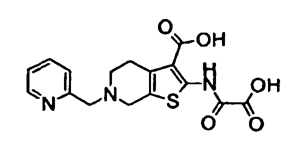

### 2-(Oxalyl-amino)-6-pyridin-2-ylmethyl-4,5,6,7-tetrahydro-thieno[2,3-c]pyridine-3-carboxylic acid;

To a mixture of 2-(ethoxyoxalyl-amino)-4,5,6,7-tetrahydro-thieno[2,3-c]pyridine-3-carboxylic acid ethyl ester trifluoroacetic acid salt (1.5 g, 3.40 mmol, prepared as described in Example 8), potassium carbonate (2.4 g, 17.1 mmol), potassium iodine (100 mg) in acetone (40 ml) was added 2-picolyl chloride hydrochloride (0.61 g, 3.7 mmol). The resulting mixture was stirred at reflux temperature for 18 h., filtered and evaporated in vacuo. The residue was trituated with diethyl ether and the solid was filtered off and purified on silicagel (300 ml) using a mixture of ethyl acetate/ethanol/triethyl amine (3:1:0.4) as eluent. Pure fractions were collected and the eluent evaporated in vacuo affording 650 mg (39 %) of 2-(ethoxyoxalyl-amino)-6-pyridin-2-ylmethyl-4,5,6,7-tetrahydro-thieno[2,3-c]pyridine-3-carboxylic acid triethyl ammonium salt as a solid.

To a solution of the above triethyl ammonium salt (650 mg, 1.40 mmol) in ethanol (15 ml) was added 1N aqueous sodium hydroxide (4.1 ml, 4.1 mmol) followed by water (15 ml). The resulting reaction mixture was stirred at room temperature for 18 h. The volatiles were evaporated in vacuo and the residue dissolved in water (20 ml) and washed with diethyl ether (2 x 10 ml). To the aqueous phase was added 1N hydrochloric acid to pH = 1 and the aqueous phase was evaporated in vacuo. The residue was suspended in a mixture of 2-propanol/water (1:1, 40 ml), stirred for 1 h., the solid filtered off and washed with 2-propanol (2 x 15 ml) and dried in vacuo at 50 °C affording 181 mg (38 %) of crude title compound. The crude product (181 mg) was dissolved in a mixture of water (10 ml) and
5 N sodium hydroxide (10 ml) and washed with diethyl ether (2 x 10 ml). The aqueous phase was acidified to pH = 3 with 1N hydrochloric acid and the precipitate filtered off and washed with water (3 x 20 ml), dried in vacuo at 50 °C for 18 h which afforded 51 mg (11%) of the title compound as a solid.
M.p.: 238 - 244 °C
Calculated for C₁₆H₁₅N₃O₅S, 2.5 x H₂O;
C, 47.29 %; H, 4.96 %; N, 10.34 %. Found: ,
C, 47.43 %; H, 4.84 %; N, 10.00 %.

By a similar procedure as described in Example 7 the following compounds were prepared.

### EXAMPLE 8

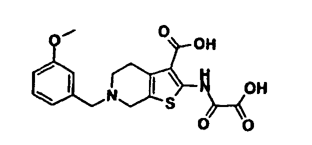

### 6-(3-Methoxy-benzyl)-2-(oxalyl-amino)-4,5,6,7-tetrahydro-thieno[2,3-c]pyridine-3-carboxylic acid;

M.p.: 233 - 237 °C
Calculated for C₁₈H₁₈N₂O₆S, 1 x H₂O;
C, 52.93 %; H, 4.94 %; N, 6.86 %. Found:
C, 52.79 %; H, 4.99 %; N, 6.42 %.

### EXAMPLE 9

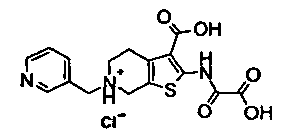

### 2-(Oxalyl-amino)-6-pyridin-3-ylmethyl-4,5,6,7-tetrahydro-thieno[2,3-c]pyridine-3-carboxylic acid, hydrochloride;

M.p.: 234 - 238 °C
Calculated for C₁₆H₁₅N₃O₅S, 1 x HCl, 0.5 x H₂O;
C, 47.24 %; H, 4.21 %; N, 10.33 %. Found:
C, 47.35 %; H, 4.10 %; N, 10.35 %.

### EXAMPLE 10

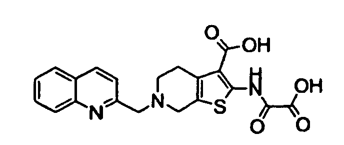

### 2-(Oxalyl-amino)-6-quinolin-2-ylmethyl-4,5,6,7-tetrahydro-thieno[2,3-c]pyridine-3-carboxylic acid;

M.p.: > 250 °C
Calculated for C₂₀H₁₇N₃O₅S, 1 x H₂O;
C, 55.95 %; H, 4.22 %; N, 9.61 %. Found:
C, 55.94 %; H, 4.46 %; N, 9.78 %.

### EXAMPLE 11

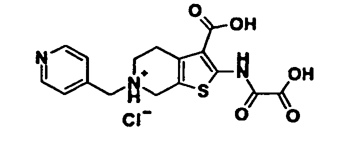

### 2-(Oxalyl-amino)-6-pyridin-4-ylmethyl-4,5,6,7-tetrahydro-thieno[2,3-c]pyridine-3-carboxylic acid, hydrochloride;

M.p.: 230 - 235 °C
Calculated for C₁₆H₁₅N₃O₅S, 1 x HCl, 1 x H₂O;
C, 46.21 %; H, 4.36 %; N, 10.10 %. Found:
C, 45.82 %; H, 4.42 %; N, 10.02 %.

By a similar procedure as described in Example 1 the following compounds were prepared.

### EXAMPLE 12

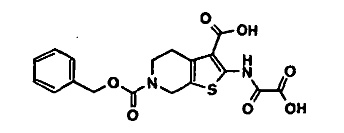

### 2-(Oxalyl-amino)-4,7-dihydro-5H-thieno[2,3-c]pyridine-3,6-dicarboxylic acid 6-benzyl ester

M.p.: >250 °C
Calculated for C₁₈H₁₆N₂O₇S;
C, 53.46 %; H, 3.99 %; N, 6.93 %. Found:
C, 53.44 %; H, 4.15 %; N, 6.69 %.

### EXAMPLE 13

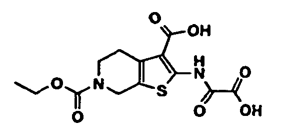

### 2-(Oxalyl-amino)-4,7-dihydro-5H-thieno[2,3-c]pyridine-3,6-dicarboxylic acid 6-ethyl ester

M.p.: 245 - 247 °C
Calculated for C₁₃H₁₄N₂O₇S;
C, 45.61 %; H, 4.12 %; N, 8.18 %. Found:
C, 45.71 %; H, 4.31 %; N, 7.86 %.

### EXAMPLE 14

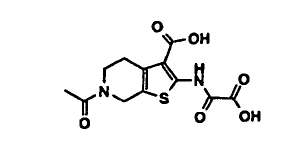

### 6-Acetyl-2-(oxalyl-amino)-4,5,6,7-tetrahydro-thieno[2,3-c]pyridine-3-carboxylic acid

M.p.: 242 - 244 °C
Calculated for C₁₂H₁₂N₂O₆S, 0.25 x H₂O;
C, 45.50 %; H, 3.98 %; N, 8.84 %. Found:
C, 45.64 %; H, 3.97 %; N, 8.51 %.

### EXAMPLE 15

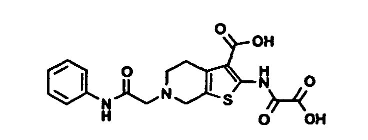

### 2-(Oxalyl-amino)-6-phenylcarbamoylmethyl-4,5,6,7-tetrahydro-thieno[2,3-c]pyridine-3-carboxylic acid

M.p.: 244 - 246 °C
Calculated for C₁₈H₁₇N₃O₆S, 1 x H₂O;
C, 51.30 %; H, 4.54 %; N, 9.97 %. Found:
C, 51.08 %; H, 4.52 %; N, 9.63 %.

## Claims

1. A compound of Formula 1 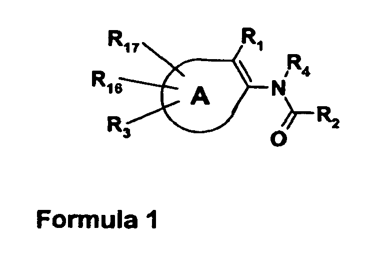 wherein
A is together with the double bond in Formula 1 4,5,6,7-tetrahydro-thieno[2,3-*b*]pyridyl, 4,5,6,7-tetrahydro-thieno[2,3-*c*]pyridyl, 4,5,6,7-tetrahydro-thieno[3,2-*c*]pyridyl, 4,5,6,7-tetrahydro-thieno[3,2-*b*]pyridyl, or 4,5,6,7-tetrahydro-4,7-ethano-thieno[2,3-b]pyridyl;
R₁ is hydrogen, COR₅, OR₆, CF₃, nitro, SO₃H, SO₂NR₇R₈, PO(OH)₂, CH₂PO(OH)₂, CHFPO(OH)₂, CF₂PO(OH)₂, C(=NH)NH₂, NR₇R₈ or selected from the following 5-membered heterocycles: 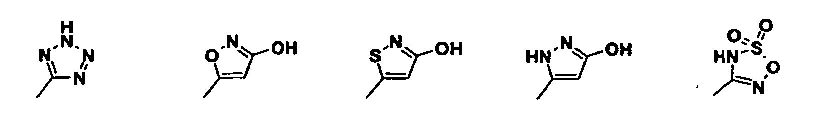 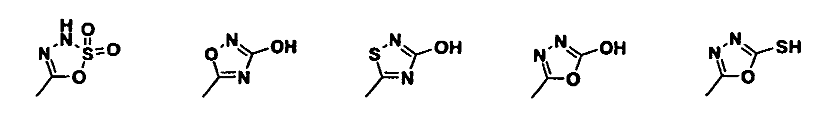 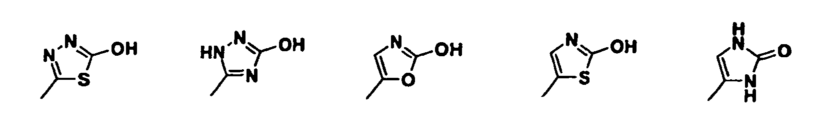 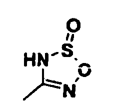 or R₁ is 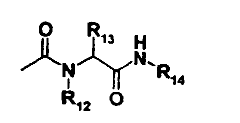 wherein R₁₂, R₁₃, and R₁₄ are independently hydrogen, C₁-C₆alkyl, aryl, arylC₁-C₆alkyl and the alkyl and aryl groups are optionally substituted;
R₂ is COR₅, OR₆, CF₃, nitro, cyano, SO₃H, SO₂NR₇R₈, PO(OH)₂, CH₂PO(OH)₂, CHFPO(OH)₂, CF₂PO(OH)₂, C(=NH)NH₂, NR₇R₈ , or selected from the following 5-membered heterocycles: 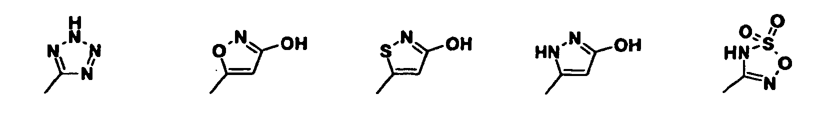 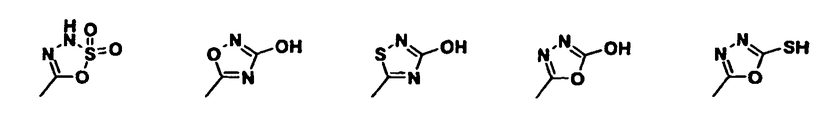 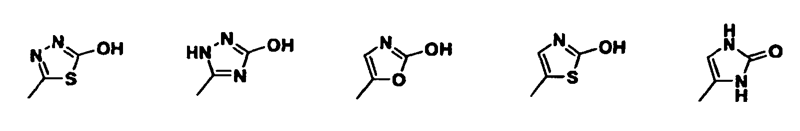 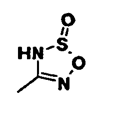 R₃, R₁₆ and R₁₇ are independently hydrogen, halo, nitro, cyano, trihalomethyl, C₁-C₆alkyl, aryl, arylC₁-C₆alkyl, hydroxy, oxo, carboxy, carboxyC₁-C₆alkyl, C₁-C₆alkyloxycarbonyl, aryloxycarbonyl, arylC₁-C₆alkyloxycarbonyl, C₁-C₆alkyloxy, C₁-C₆alkyloxyC₁-C₆alkyl, aryloxy, arylC₁-C₆alkyloxy, arylC₁-C₆alkyloxyC₁-C₆alkyl, thio, C₁-C₆alkylthio, C₁-C₆alkylthioC₁-C₆alkyl, arylthio, arylC₁-C₆alkylthio, arylC₁-C₆alkylthioC₁-C₆alkyl, NR₇R₈, C₁-C₆alkylaminoC₁-C₆alkyl, arylC₁-C₆alkylaminoC₁-C₆alkyl, di(arylC₁-C₆alkyl)aminoC₁-C₆alkyl, C₁-C₆alkylcarbonyl, C₁-C₆alkylcarbonyl-C₁-C₆alkyl, arylC₁-C₆alkylcarbonyl, arylC₁-C₆alkylcarbonylC₁-C₆alkyl, C₁-C₆alkylcarboxy, C₁-C₆alkylcarboxyC₁-C₆-alkyl, arylcarboxy, arylcarboxyC₁-C₆alkyl, arylC₁-C₆alkylcarboxy, arylC₁-C₆alkylcarboxyC₁-C₆alkyl, C₁-C₆alkyl-carbonylamino, C₁-C₆alkylcarbonylaminoC₁-C₆alkyl, -carbonylNR₇C₁-C₆alkylCOR₁₁, arylC₁-C₆alkylcarbonylamino, arylC₁-C₆alkylcarbonyl-aminoC₁-C₆alkyl, CONR₇R₈, or C₁-C₆alkylCONR₇R₈ wherein the alkyl and aryl groups are optionally substituted and R₁₁ is NR₇R₈, or C₁-C₆alkylNR₇R₈; or R₃ is 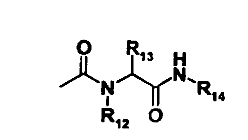 wherein R₁₂, R₁₃, and R₁₄ are independently hydrogen, C₁-C₆alkyl, aryl, arylC₁-C₆alkyl and the alkyl and aryl groups are optionally substituted;
R₄ is hydrogen, hydroxy, C₁-C₆alkyl, aryl, arylC₁-C₆alkyl, NR₇R₈, C₁-C₆alkyloxy; wherein the alkyl and aryl groups are optionally substituted;
R₅ is hydroxy, C₁-C₆alkyl, aryl, arylC₁-C₆alkyl, C₁-C₆alkyl-oxyC₁-C₆alkyloxy, aryloxy, arylC₁-C₆alkyloxy, CF₃ ; wherein the alkyl and aryl groups are optionally substituted;
R₆ is hydrogen, C₁-C₆alkyl, aryl, arylC₁-C₆alkyl; wherein the alkyl and aryl groups are optionally substituted;
R₇ and R₈ are independently selected from hydrogen, C₁-C₆alkyl, aryl, arylC₁-C₆alkyl, C₁-C₆alkylcarbonyl, arylcarbonyl, arylC₁-C₆alkylcarbonyl, C₁-C₆alkylcarboxy or arylC₁-C₆alkylcarboxy wherein the alkyl and aryl groups are optionally substituted; or R₇ and R₈ are together with the nitrogen to which they are attached forming a saturated, partially saturated or aromatic cyclic, bicyclic or tricyclic ring system containing from 3 to 14 carbon atoms and from 0 to 3 additional heteroatoms selected from nitrogen, oxygen or sulfur, the ring system can optionally be substituted with at least one C₁-C₆alkyl, aryl, arylC₁-C₆alkyl, hydroxy, oxo, C₁-C₆alkyloxy, arylC₁-C₆alkyloxy, C₁-C₆alkyloxyC₁-C₆alkyl, NR₉R₁₀ or C₁-C₆alkylaminoC₁-C₆alkyl, wherein R₉ and R₁₀ are independently selected from hydrogen, C₁-C₆alkyl, aryl, arylC₁-C₆alkyl, C₁-C₆alkylcarbonyl, arylcarbonyl, arylC₁-C₆alkylcarbonyl, C₁-C₆alkylcarboxy or arylC₁-C₆alkylcarboxy; wherein the alkyl and aryl groups are optionally substituted; or R₇ and R₈ are independently a saturated or partial saturated cyclic 5, 6 or 7 membered amine, imide or lactam;
or a salt thereof with a pharmaceutically acceptable acid or base, or any optical isomer or mixture of optical isomers, including a racemic mixture, or any tautomeric forms;
wherein alkyl represents C₁-C₆ straight chain saturated, methylene and C₂-C₆ unsaturated aliphatic hydrocarbon groups, C₁-C₆ branched saturated and C₂-C₆ unsaturated aliphatic hydrocarbon groups, C₃-C₆ cyclic saturated and C₅-C₆ unsaturated aliphatic hydrocarbon groups, and C₁-C₆ straight chain or branched saturated and C₂-C₆ straight chain or branched unsaturated aliphatic hydrocarbon groups substituted with C₃-C₆ cyclic saturated and unsaturated aliphatic hydrocarbon groups having the specified number of carbon atoms;
alkylamino represents one or two alkyl groups as defined above having the indicated number of carbon atoms attached through an amine bridge, wherein the two alkyl groups may be taken together with the nitrogen to which they are attached forming a saturated, partially saturated or aromatic cyclic, bicyclic or tricyclic ring system containing 3 to 14 carbon atoms and 0 to 3 additional heteroatoms selected from nitrogen, oxygen or sulphur;
aryl represents an unsubstituted, mono-, di- or trisubstituted monocyclic, polycyclic, biaryl and heterocyclic aromatic group covalently attached at any ring position capable of forming a stable covalent bond;
with the proviso that when A together with the double bond in Formula 1 is 4,5,6,7-tetrahydro-thieno[2,3-*c*]pyridyl and R₁ is cyano, then R₂ is not COR₅ wherein R₅ is hydroxy, C₁-C₆-alkyloxy, phenyloxy, or benzyloxy.

2. A compound according to claim 1 wherein A is 4,5,6,7-tetrahydro-thieno[2,3-*b*]pyridyl.

3. A compound according to claim 2 wherein R₁ and R₂ are COR₅ and R₄ is hydrogen; wherein R₅ is defined as above.

4. A compound according to claim 2 wherein R₁ is 5-tetrazolyl and R₂ is COR₅; wherein R₅ is defined as above.

5. A compound according to claim 2 wherein R₁ and R₂ are COOH and R₄ is hydrogen.

6. A compound according to claim 1 wherein A is 4,5,6,7-tetrahydro-thieno[2,3-*c*]pyridyl.

7. A compound according to claim 6 wherein R₁ and R₂ are COR₅ and R₄ is hydrogen; wherein R₅ is defined as above.

8. A compound according to claim 6 wherein R₁ is 5-tetrazolyl and R₂ is COR₅; wherein R₅ is defined as above.

9. A compound according to claim 6 wherein R₁ and R₂ are COOH and R₄ is hydrogen.

10. A compound according to claim 1 wherein A is 4,5,6,7-tetrahydro-thieno[3,2-*c*]pyridyl.

11. A compound according to claim 10 wherein R₁ and R₂ are COR₅ and R₄ is hydrogen; wherein R₅ is defined as above.

12. A compound according to claim 10 wherein R₁ is 5-tetrazolyl and R₂ is COR₅; wherein R₅ is defined as above.

13. A compound according to claim 10 wherein R₁ and R₂ are COOH and R₄ is hydrogen.

14. A compound according to claim 1 wherein A is 4,5,6,7-tetrahydro-thieno[3,2-*b*]pyridyl.

15. A compound according to claim 14 wherein R₁ and R₂ are COR₅ and R₄ is hydrogen; wherein R₅ is defined as above.

16. A compound according to claim 14 wherein R₁ is 5-tetrazolyl and R₂ is COR₅; wherein R₅ is defined as above.

17. A compound according to claim 14 wherein R₁ and R₂ are COOH and R₄ is hydrogen.

18. A compound according to claim 1 wherein A is 4,5,6,7-tetrahydro-4,7-ethano-thieno[2,3-b]pyridyl.

19. A compound according to claim 18 wherein R₁ and R₂ are COR₅ and R₄ is hydrogen; wherein R₅ is defined as above.

20. A compound according to claim 18 wherein R₁ is 5-tetrazolyl and R₂ is COR₅; wherein R₅ is defined as above.

21. A compound according to claim 18 wherein R₁ and R₂ are COOH and R₄ is hydrogen.

22. A compound selected from the following:
6-Benzoyl-2-(oxalyl-amino)-4,5,6,7-tetrahydro-thieno[2,3-*c*]pyridine-3-carboxylic acid;
6-Benzyl-2-(oxalyl-amino)-4,5,6,7-tetrahydro-thieno[2,3-*c*]pyridine-3-carboxylic acid;
6-Methyl-2-(oxalyl-amino)-4,5,6,7-tetrahydro-thieno[2,3-*c*]pyridine-3-carboxylic acid;
2-(Oxalyl-amino)-6-phenethyl-4,5,6,7-tetrahydro-thieno[2,3-c]pyridine-3-carboxylic acid;
5-Benzoyl-2-(oxalyl-amino)-4,5,6,7-tetrahydro-thieno[3,2-*c*]pyridine-3-carboxylic acid;
5-Benzyl-2-(oxalyl-amino)-4,5,6,7-tetrahydro-thieno[3,2-*c*]pyridine-3-carboxylic acid;
5-Methyl-2-(oxalyl-amino)-4,5,6,7-tetrahydro-thieno[3,2-*c*]pyridine-3-carboxylic acid;
2-(Oxalyl-amino)-5-phenethyl-4,5,6,7-tetrahydro-thieno[3,2-*c*]pyridine-3-carboxylic acid;
2-(Oxalyl-amino)-4,5,6,7-tetrahydro-4,7-ethano-thieno[2,3-b]pyridine-3-carboxylic acid;
2-(Oxalyl-amino)-6-pyridin-2-ylmethyl-4,5,6,7-tetrahydro-thieno[2,3-c]pyridine-3-carboxylic acid;
2-(Oxalyl-amino)-4,5,6,7-tetrahydro-thieno[2,3-c]pyridine-3-carboxylic acid;
6-(3-Methoxy-benzyl)-2-(oxalyl-amino)-4,5,6,7-tetrahydro-thieno[2,3-c]pyridine-3-carboxylic acid;
2-(Oxalyl-amino)-6-pyridin-3-ylmethyl-4,5,6,7-tetrahydro-thieno[2,3-c]pyridine-3-carboxylic acid;
2-(Oxalyl-amino)-6-pyridin-4-ylmethyl-4,5,6,7-tetrahydro-thieno[2,3-c]pyridine-3-carboxylic acid;
2-(Oxalyl-amino)-6-quinolin-2-ylmethyl-4,5,6,7-tetrahydro-thieno[2,3-c]pyridine-3-carboxylic acid;
6-Acetyl-2-(oxalyl-amino)-4,5,6,7-tetrahydro-thieno[2,3-c]pyridine-3-carboxylic acid;
2-(Oxalyl-amino)-6-phenylcarbamoylmethyl-4,5,6,7-tetrahydro-thieno[2,3-c]pyridine-3-carboxylic acid;
or a pharmaceutically acceptable salt thereof.

23. Compounds according to any one of the preceding claims which act as inhibitors or modulators of Protein Tyrosine Phosphatases.

24. A pharmaceutical composition suitable for treating type I diabetes, type II diabetes, impaired glucose tolerance, insulin resistance or obesity comprising a compound according to any of the claims 1 to 22 or a pharmaceutical acceptable salt thereof with a pharmaceutically acceptable acid or base, or any optical isomer or mixture of optical isomers, including a racemic mixture, or any tautomeric forms together with one or more pharmaceutically acceptable carriers or diluents.

## Patentansprüche

1. Verbindung der Formel 1 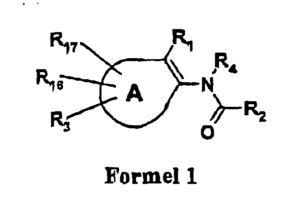 wobei
A zusammen mit einer Doppelbindung in Formel 1 4,5,6,7-Tetrahydrothieno[2,3-*b*]pyridyl, 4,5,6,7-Tetrahydrothieno[2,3-*c*]pyridyl, 4,5,6,7-Tetrahydrothieno[3,2-*c*]pyridyl, 4,5,6,7-Tetrahydrothieno[3,2-*b*]pyridyl oder 4,5,6,7-Tetrahydro-4,7-ethanonthieno[2,3-b]pyridyl ist;
R₁ Wasserstoff, COR₅, OR₆, CF₃, nitro, SO₃H, SO₂NR₇R₈, PO(OH)₂, CH₂PO(OH)₂, CHFPO(OH)₂, CF₂PO(OH)₂, C(=NH)NH₂, NR₇R₈ ist oder ausgewählt ist aus den folgenden 5-gliedrigen Heterocyclen: 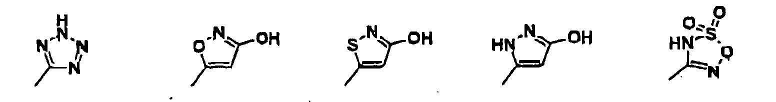 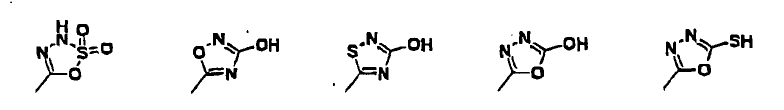 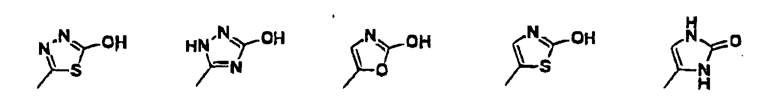 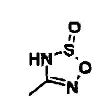 oder R₁ 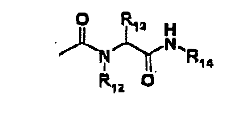 ist, wobei R₁₂, R₁₃ und R₁₄ unabhängig Wasserstoff, C₁-C₆-Alkyl, Aryl, Aryl-C₁-C₆-alkyl sind und die Alkyl- und Arylgruppen wahlweise substituiert sind;
R₂ COR₅, OR₆, CF₃, Nitro, Cyano, SO₃H, SO₂NR₇R₈, PO(OH)₂, CH₂PO(OH)₂, CHFPO(OH)₂, CF₂PO(OH)₂, C(=NH)NH₂, NR₇R₈ ist oder ausgewählt ist aus den folgenden 5-gliedrigen Heterocyclen: 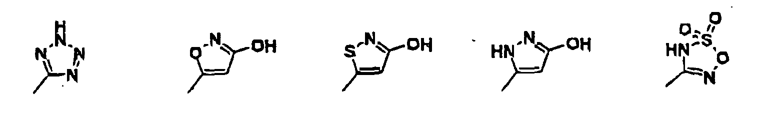 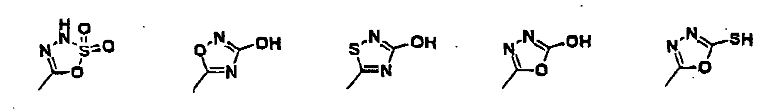 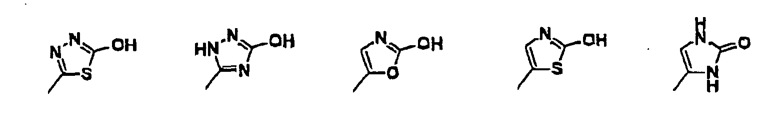 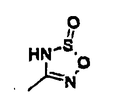 R₃, R₁₆ und R₁₇ unabhängig Wasserstoff, Halogen, Nitro, Cyano, Trihalogenmethyl, C₁-C₆-Alkyl, Aryl, Aryl-C₁-C₆-alkyl, Hydroxy, Oxo, Carboxy, Carboxy-C₁-C₆-alkyl, C₁-C₆-alkyloxycarbonyl, Aryloxyoarbonyl, Aryl-C₁-C₆-alkyloxycarbonyl, C₁-C₆-Alkyloxy, C₁-C₆-Alkyloxy-C₁-C₆-alkyl, Aryloxy, Aryl-C₁-C₆-alkyloxy, Aryl-C₁-C₆-alkyloxy-C₁-C₆-alkyl, Thio, C₁-C₆-Alkylthio, C₁-C₆-Alkylthio-C₁-C₆-alkyl, Arylthio, Aryl-C₁-C₆-alkylthio, Aryl-C₁-C₆-alkylthio-C₁-C₆-alkyl, NR₇R₈, C₁-C₆-Alkylamino-C₁-C₆-alkyl, Aryl-C₁-C₆-alkylamino-C₁-C₆-alkyl, Di(arylC₁-C₆alkyl)amino-C₁-C₆-alkyl, C₁-C₆-alkylcarbonyl, C₁-C₆-Alkylcarbonyl-C₁-C₆-alkyl, Aryl-C₁-C₆-alkylcarbonyl, Aryl-C₁-C₆-alkylcarbonyl-C₁-C₆-alkyl, C₁-C₆-Alkylcarboxy, C₁-C₆-Alkylcarboxy-C₁-C₆-alkyl, Arylcarboxy, Arylcarboxy-C₁-C₆-alkyl, ArylC₁-C₆-alkylcarboxy, Aryl-C₁-C₆-alkylcarboxy-C₁-C₆-alkyl, C₁-C₆-Alkylcarbonylamino, C₁-C₆-Alkylcarbonylamino-C₁-C₆-alkyl, -Carbonyl-NR₇C₁-C₆-alkyl-COR₁₁, Aryl-C₁-C₆-alkylcarbonylamino, Aryl-C₁-C₆-alkylcarbonylamino-C₁-C₆-alkyl, CONR₇R₈ oder C₁-C₆-Alkyl-CONR₇R₈ sind, wobei die Alkyl- und Arylgruppen wahlweise substituiert sind und R₁₁ NR₇R₈ oder C₁-C₆-Alkyl-NR₇R₈ ist; oder R₃ 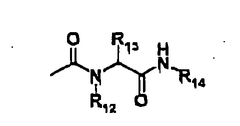 ist, wobei R₁₂, R₁₃ und R₁₄ unabhängig Wasserstoff, C₁-C₆-Alkyl, Aryl, Aryl-C₁-C₆-alkyl sind und die Alkyl- und Arylgruppen wahlweise substituiert sind;
R₄ Wasserstoff, Hydroxy, C₁-C₆-Alkyl, Aryl, Aryl-C₁-C₆-alkyl, NR₇R₈, C₁-C₆-Alkyloxy ist; wobei die Alkyl- und Arylgruppen wahlweise substituiert sind;
R₅ Hydroxy, C₁-C₆-Alkyl, Aryl, Aryl-C₁-C₆-alkyl, C₁-C₆-Alkyloxy, C₁-C₆-Alkyloxy-C₁-C₆-alkyloxy, Aryloxy, Aryl-C₁-C₆-alkyloxy, CF₃ ist; wobei die Alkyl- und Arylgruppen wahlweise substituiert sind;
R₆ Wasserstoff, C₁-C₆-Alkyl, Aryl, Aryl-C₁-C₆-alkyl ist; wobei die Alkyl- und Arylgruppen wahlweise substituiert sind;
R₇ und R₈ unabhängig ausgewählt sind aus Wasserstoff, C₁-C₆-Alkyl, Aryl, Aryl-C₁-C₆-alkyl, C₁-C₆-Alkylcarbonyl, Arylcarbonyl, Aryl-C₁-C₆-alkylcarbonyl, C₁-C₆-Alkylcarboxy oder Aryl-C₁-C₆-alkylcarboxy, wobei die Alkyl- und Arylgruppen wahlweise substituiert sind; oder
R₇ und R₈ zusammen mit dem Stickstoffatom, an welchem sie angelagert sind, ein gesättigtes, teilweise gesättigtes oder aromatisches cyclisches, bicyclisches oder tricyclisches Ringsystem bilden, das 3 bis 14 Kohlenstoffatome und 0 bis 3 zusätzliche Heteroatome, ausgewählt aus Stickstoff, Sauerstoff und Schwefel, enthält, wobei das Ringsystem wahlweise mit mindestens einem C₁-C₆-Alkyl, Aryl, Aryl-C₁-C₆-alkyl, Hydroxy, Oxo, C₁-C₆-Alkyloxy, Aryl-C₁-C₆-alkyloxy, C₁-C₆-Alkyloxy-C₁-C₆-alkyl, NR₉R₁₀ oder C₁-C₆-Alkylamino-C₁-C₆-alkyl, wobei R₉ und R₁₀ unabhängig ausgewählt sind aus Wasserstoff, C₁-C₆-Alkyl, Aryl, Aryl-C₁-C₆-alkyl, C₁-C₆-Alkylcarbonyl, Arylcarbonyl, Aryl-C₁-C₆-alkylcarbonyl, C₁-C₆-Alkylcarboxy oder Aryl-C₁-C₆-alkylcarboxy; wobei die Alkyl- und Arylgruppen wahlweise substituiert sind; oder
R₇ und R₈ unabhängig ein gesättigtes oder teilweise gesättigtes cyclisches 5-, 6- oder 7-gliedriges Amin, Imid oder Lactam sind;
oder ein Salz davon mit einer pharmazeutisch verträglichen Säure oder Base, oder ein beliebiges optisches Isomer oder Gemisch aus optischen Isomeren, einschließlich eines racemischen Gemisches, oder beliebige tautomere Formen;
wobei Alkyl geradkettige gesättigte C₁-C₆-Methylen- und ungesättigte aliphatische C₂-C₆-Kohlenwasserstoffgruppen, verzweigte gesättigte C₂-C₆-und ungesättigte aliphatische C₂-C₆-Kohlenwasserstoffgruppen, cyclische gesättigte C₃-C₆- und ungesättigte aliphatische C₅-C₆-Kohlenwasserstoffgruppen und geradkettige C₁-C₈- oder verzweigte gesättigte und geradkettige oder verzweigte ungesättigte aliphatische C₂-C₈-Kohlenwasserstoffgruppen, die mit cyclischen gesättigten und ungesättigten aliphatischen C₃-C₆-Kohlenwasserstoffgruppen mit der spezifizierten Anzahl an Kohlenstoffatomen substituiert sind, darstellt;
Alkylamino eine oder zwei wie vorstehend definierte Alkylgruppen mit der angegebenen Anzahl an durch eine Aminbrücke angelagerten Kohlenstoffatomen darstellt, wobei die beiden Alkylgruppen zusammen mit dem Stickstoffatom, an welchem sie angelagert sind ein gesättigtes, teilweise gesättigtes oder aromatisches cyclisches, bicyclisches oder tricyclisches Ringsystem bilden, das 3 bis 14 Kohlenstoffatome und 0 bis 3 zusätzliche Heteroatome, ausgewählt aus Stickstoff, Sauerstoff oder Schwefel, enthält;
Aryl eine unsubstituierte, mono-, di- oder trisubstituierte monocyclische, polycyclische, Biaryl- oder heterocyclische aromatische Gruppe darstellt, die kovalent an eine beliebige Ringposition angelagert ist, die eine stabile kovalente Bindung bilden kann;
mit der Maßgabe, dass, wenn A zuusammen mit der Doppelbindung in Formel 1 4,5,6,7-Tetrahydrothieno[2,3-c]pyridyl ist und R₁ Cyano ist, R₂ dann nicht COR₅ ist, wobei R⁵ Hydroxy, C₁-C₆-Alkyloxy, Phenyloxy oder Benzyloxy ist.

2. Verbindung nach Anspruch 1, wobei A 4,5,6,7-Tetrahydrothieno[2,3-b]pyridyl ist.

3. Verbindung nach Anspruch 2, wobei R₁ und R₂ COR₅ ist und R₄ Wasserstoff ist; wobei R₅ wie vorstehend definiert ist.

4. Verbindung nach Anspruch 2, wobei R₁ 5-Tetrazolyl und R₂ COR₅ ist, wobei R₅ wie vorstehend definiert ist.

5. Verbindung nach Anspruch 2, wobei R₁ und R₂ COOH sind und R₄ Wasserstoff ist.

6. Verbindung nach Anspruch 1, wobei A 4,5,6,7-Tetrahydrothieno[2,3-c]pyridyl ist.

7. Verbindung nach Anspruch 6, wobei R₁ und R₂ COR₅ ist und R₄ Wasserstoff ist; wobei R₅ wie vorstehend definiert ist.

8. Verbindung nach Anspruch 6, wobei R₁ 5-Tetrazolyl und R₂ COR₅ ist, wobei R₅ wie vorstehend definiert ist.

9. Verbindung nach Anspruch 6, wobei R₁ und R₂ COOH sind und R₄ Wasserstoff ist.

10. Verbindung nach Anspruch 1, wobei A 4,5,6,7-Tetrahydrothieno[3,2-c]pyridyl ist.

11. Verbindung nach Anspruch 10, wobei R₁ und R₂ COR₅ ist und R₄ Wasserstoff ist; wobei R₅ wie vorstehend definiert ist.

12. Verbindung nach Anspruch 10, wobei R₁ 5-Tetrazolyl und R₂ COR₅ ist, wobei R₅ wie vorstehend definiert ist.

13. Verbindung nach Anspruch 10, wobei R₁ und R₂ COOH sind und R₄ Wasserstoff ist.

14. Verbindung nach Anspruch 1, wobei A 4,5,6,7-Tetrahydrothieno[3,2-b]pyridyl ist.

15. Verbindung nach Anspruch 14, wobei R₁ und R₂ COR₅ ist und R₄ Wasserstoff ist; wobei R₅ wie vorstehend definiert ist.

16. , Verbindung nach Anspruch 14, wobei R₁ 5-Tetrazolyl und R₂ COR₅ ist, wobei R₅ wie vorstehend definiert ist.

17. Verbindung nach Anspruch 14, wobei R₁ und R₂ COOH sind und R₄ Wasserstoff ist

18. Verbindung nach Anspruch 1, wobei A 4,5,6,7-Tetrahydro-4,7-ethanothieno[2,3-b]pyridyl ist.

19. Verbindung nach Anspruch 18, wobei R₁ und R₂ COR₅ ist und R₄ Wasserstoff ist; wobei R₅ wie vorstehend definiert ist.

20. Verbindung nach Anspruch 18, wobei R₁ 5-Tetrazolyl und R₂ COR₅ ist, wobei R₅ wie vorstehend definiert ist,

21. Verbindung nach Anspruch 18, wobei R₁ und R₂ COOH sind und R₄ Wasserstoff ist.

22. Verbindung, aus gewählt aus
6-Benzoyl-2-(oxalylamino)-4,5,6,7-tetrahydrothieno[2,3-c]pyridin-3-carbonsäure;
6-Benzyl-2-(oxalylamino)-4,5,6,7-tetrahydrothieno[2,3-c]pyridin-3-carbonsäure;
6-Methyl-2-(oxalylamino)-4,5,6,7-tetrahydrothieno[2,3-c]pyridin-3-carbonsäure;
2-(Oxalylamino)-6-phenethyl-4,5,6,7-tetrahydrothieno[2,3-c]pyridin-3-carbonsäure;
5-Benzoyl-2-(oxalylamino)-4,5,6,7-tetrahydrothieno[3,2-c]pyridin-3-carbonsäure;
5-Benzyl-2-(oxalylamino)-4,5,6,7-tetrahydrothieno[3,2-c]pyridin-3-carbonsäure;
5-Methyl-2-(oxalylamino)-4,5,6,7-tetrahydrothieno[3,2-c]pyridin-3-carbonsäure;
2-(Oxalylamino)-5-phenethyl-4,5,6,7-tetrahydrothieno[3,2-c]pyridin-3-carbonsäure;
2-(Oxalylamino)-4,5,6,7-tetrahydro-4,7-ethanthieno[2,3-b]pyridin-3-carbonsäute;
2-(Oxalylamino)-6-pyridin-2-ylmethyl-4,5,6,7-tetrahydrothieno[2,3-c]pyridin-3-carbonsäure;
2-(Oxalylamino)-4,5,6,7-tetrahydrothieno[2,3-c]pyridin-3-carbonsäure;
6-(3-Methoxybenayl)-2-(oxalylamino)-4,5,6,7-tetrahydrothieno[2,3-c]pyridin-3-carbonsäure;
2-(Oxalylamino)-6-pyridin-3-ylmethyl-4,5,6,7-tetrahydrothieno[2,3-c]pyridin-3-carbonsäure;
2-(Oxalylamino)-6-pyridin-4-ylmethyl-4,5,6,7-tetrahydrothieno[2,3-c]pyridin-3-carbonsäure;
2-(Oxalylamino)-6-chinolin-2-ylmethyl-4,5,6,7-tetrahydrothieno[2,3-c]pyridin-3-carbonsäure;
6-Acetyl-2-(oxalylamino)-6-pyridin-3-ylmethyl-4,5,6,7-tetrahydrothieno[2,3-c]pyridin-3-carbonsäure;
2-(Oxalylamino)-6-phenylcarbonylmethyl-4,5,6,7-tetrahydrothieno[2,3-c]pyridin-3-carbonsäure;
oder einem pharmazeutisch verträglichen Salz davon.

23. Verbindungen nach einem der vorangehenden Ansprüche, die als Hemmstoffe oder Modulatoren für Protein-Tyrosin-Phosphatasen wirken.

24. Arzneimittel, das zur Behandlung von Diabetes Typ I, Diabetes Typ II, beeinträchtigter Glucoseverträglichkeit, Insulinresistenz oder Fettsucht geeignet ist, umfassend eine Verbindung nach einem der Ansprüche 1 bis 22 oder ein pharmazeutisch verträgliches Salz davon mit einer pharmazeutisch verträglichen Säure oder Base, oder ein beliebiges optisches Isomer oder Gemisch aus optischen Isomeren, einschließlich eines racemischen Gemisches, oder beliebige tautomere Formen zusammen mit einem oder mehreren pharmazeutisch verträglichen Trägem oder Verdünnungsmitteln.

## Revendications

1. Composé de formule 1 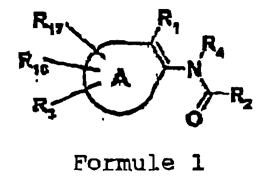 dans laquelle
A, avec la double liaison de la formule 1, est le groupe 4,5,6,7-tétrahydro-thiéno[2,3-b]pyridyle, 4,5,6,7-tétrahydro-thiéno[2,3-c]pyridyle, 4,5,6,7-tétrahydro-thiéno[2,2-c]pyridyle, 4,5,6,7-tétrahydro-thiéno[3,2-b]pyridyle ou 4,5,6,7-tétrahydro-4,7-éthanothiéno[2,3-b]pyridyle ;
R₁ est un atome d'hydrogène, COR₅, OR₆, CF₃, le groupe nitro, SO₃H, SO₂NR₇R₈, PO(OH)₂, CH₂PO(OH)₂, CHFPO(OH)₂), CF₂PO(OH)₂, C(=NH)NH₂, NR₇R₈, ou est choisi parmi les radicaux hétérocycliques à 5 chaînons suivants : 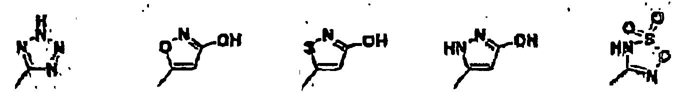 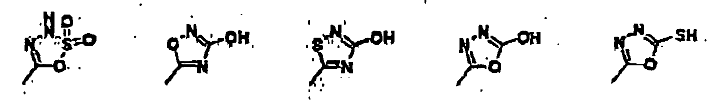 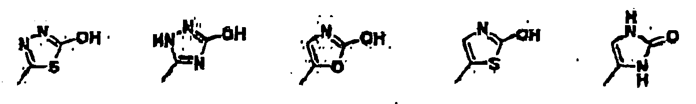 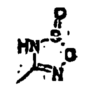 ou bien R₁ est 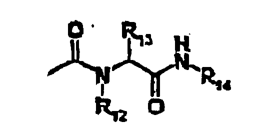 où R₁₂, R₁₃ et R₁₄ représentent chacun indépendamment des autres un atome d'hydrogène ou un groupe alkyle en C₁-C₆, aryle, aryl(alkyle en C₁-C₆), et les groupes alkyle et aryle sont éventuellement substitués ;
R₂ est COR₅, OR₆, CF₃, le groupe nitro, cyano, SO₃H, SO₂NR₇R₈, PO(OH)₂, CH₂PO(OH)₂, CHFFO(OH)₂, CF₂FO(OH)₂, C(=NH)NH₂, NR₇R₈, ou bien est choisi parmi les radicaux hétérocycliques à 5 chaînons suivants : 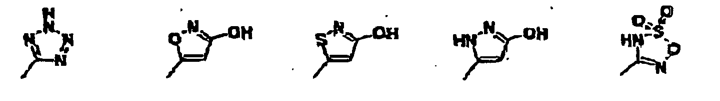 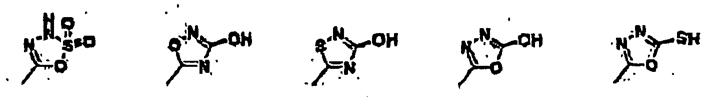 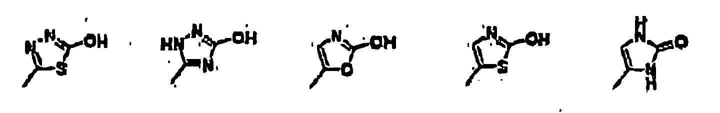 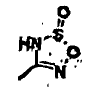 R₃, R₁₆ et R₁₇ représentent chacun indépendamment des autres un atome d'hydrogène ou un groupe halogéno, nitro, cyano, trihalogénométhyle, alkyle en C₁-C₆, aryle, aryl-(alkyle en C₁-C₆), hydroxy, oxo, carboxy, carboxy(alkyle en C₁-C₆), (alkyle en C₁-C₆)oxycarbonyle, aryloxycarbonyle, aryl(alkyle en C₁-C₆)oxycarbonyle, alkyloxy en C₁-C₆, (alkyle en C₁-C₆)oxy(alkyle en C₁-C₆), aryloxy, aryl(alkyle en C₁-C₆)oxy, aryl(alkyle en C₁-C₆)oxy(alkyle en C₁-C₆), thio, alkylthio en C₁-C₆, (alkyle en C₁-C₆)thio(alkyle en C₁-C₆), arylthio, aryl(alkyle en C₁-C₆)thio, aryl(alkyle en C₁-C₆)thio(alkyle en C₁-C₆), NR₇R₈, (alkyle en C₁-C₆)-amino (alkyle en C₁-C₆), aryl (alkyle en C₁-C₆)amino (alkyle en C₁-C₆), di(aryl(alkyle en C₁-C₆))amino(alkyle en C₁-C₆), (alkyle en C₁-C₆)carbonyle, (alkyle en C₁-C₆)carbonyl-(alkyle en C₁-C₆), aryl (alkyle en C₁-C₆)carbonyle, aryl-(alkyle en C₁-C₆)carbonyl(alkyle en C₁-C₆), (alkyle en C₁-C₆) carboxy, (alkyle en C₁-C₆)carboxy(alkyle en C₁-C₆), arylcarboxy, arylcarboxy(alkyle en C₁-C₆), aryl(alkyle en C₁-C₆)carboxy, aryl(alkyle en C₁-C₆)carboxy(alkyle en C₁-C₆), (alkyle en C₁-C₆)carbonylamino, (alkyle en C₁-C₆)-carbonylamino(alkyle en C₁-C₆), -carbonylNR₇(alkyle en C₁-C₆)COR₁₁, aryl (alkyle en C₁-C₆)carbonylamino, aryl (alkyle en C₁-C₆)carbonylamino(alkyle en C₁-C₆), CONR₇R₈ ou (alkyle en C₁-C₆)CONR₇R₈, où les groupes alkyle et aryle sont éventuellement substitues, et R₁₁ est NR₇R₈ ou un radical (alkyle en C₁-C₆)NR₇R₈ ; ou bien R₃ est 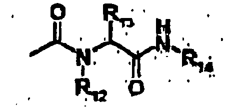 où R₁₂, R₁₃ et R₁₄ représentent chacun indépendamment des autres un atome d'hydrogène ou un groupe alkyle en C₁-C₆, aryle, aryl(alkyle en C₁-C₆), et les groupes alkyle et aryle sont éventuellement substitués ;
R₄ est un atome d'hydrogène ou un groupe hydroxy, alkyle en C₁-C₆, aryle, aryl(alkyle en C₁-C₆), NR₇R₈, alkyloxy en C₁-C₆ ; où les groupes alkyle et aryle sont éventuellement substitués ;
R₅ est un groupe hydroxy, alkyle en C₁-C₆, aryle, aryl(alkyle en C₁-C₆), (alkyle en C₁-C₆)oxy(alkyle en C₁-C₆)oxy, aryloxy, aryl(alkyle en C₁-C₆)oxy, CF₃ ; où les groupes alkyle et aryle sont éventuellement substitués ;
R₆ est un atome d'hydrogène ou un groupe alkyle en C₁-C₆, aryle, aryl(alkyle en C₁-C₆) ; où les groupes alkyle et aryle sont éventuellement substitués ;
R₇ et R₈ sont choisis d'une manière indépendante parmi l'atome d'hydrogène et les groupes alkyle en C₁-C₆, aryle, aryl(alkyle en C₁-C₆), (alkyle en C₁-C₆)carbonyle, arylcarbonyle, aryl(alkyle en C₁-C₆)carbonyle, (alkyle en C₁-C₆)carboxy ou aryl(alkyle en C₁-C₆)carboxy, où les groupes alkyle et aryle sont éventuellement substitués ; ou bien R₇ et R₈, avec l'atome d'azote auquel ils sont liés, forment un système cyclique, bicyclique ou tricyclique saturé, partiellement saturé ou aromatique, contenant de 3 à 14 atomes de carbone et de 0 à 3 hétéroatomes additionnels choisis parmi l'azote, l'oxygène ou le soufre, le système cyclique pouvant en option être substitué par au moins un substituant alkyle en C₁-C₆, aryle, aryl(alkyle en C₁-C₆), hydroxy, oxo, alkyloxy en C₁-C₆, aryl(alkyle en C₁-C₆)oxy, (alkyle en C₁-C₆)oxy(alkyle en C₁-C₆), NR₉R₁₀ ou (alkyle en C₁-C₆)amino (alkyle en C₁-C₆), où R₉ et R₁₀ sont choisis indépendamment l'un de l'autre parmi l'atome d'hydrogène et les groupes alkyle en C₁-C₆, aryle, aryl(alkyle en C₁-C₆), (alkyle en C₁-C₆)carbonyle, arylcarbonyle, aryl(alkyle en C₁-C₆)carbonyle, (alkyle en C₁-C₆)carboxy ou aryl(alkyle en C₁-C₆)carboxy ; où les groupes alkyle et aryle sont éventuellement substitués ; ou encore R₇ et R₈ représentent indépendamment une amine, un imide ou un lactame cyclique à 5, 6 ou 7 chaînons, saturé ou partiellement saturé ;
ou l'un de ses sels avec un acide ou une base acceptable d'un point de vue pharmaceutique, ou tout isomère optique ou mélange d'isomères optiques, y compris un mélange racémique, ou toutes formes tautomères ;
où alkyle représente une chaîne droite en C₁-C₆ saturée des groupes méthylène et hydrocarbonés aliphatiques insaturés en C₂-C₆, des groupes hydrocarbonés aliphatiques, saturés ramifiés en C₁-C₆ et insaturés en C₂-C₆, des groupes hydrocarbonés aliphatiques saturés cycliques en C₃-C₆ et insaturés en C₅-C₆, et des groupes hydrocarbonés aliphatiques à chaîne droite ou saturés et ramifiés en C₁-C₆, et à chaîne droite ou insaturés et ramifiés en C₂-C₆, substitués par des groupes hydrocarbonés aliphatiques cycliques saturés et insaturés en C₃-C₆, ayant le nombre spécifié d'atomes de carbone ;
alkylamino représente un ou deux groupes alkyle tels que définis ci-dessue, ayant le nombre indiqué d'atomes de carbone fixés par l'intermédiaire d'un pont amine, où les deux groupes alkyle peuvent être pris ensemble avec l'atome d'azote auquel ils sont liés pour former un système cyclique, bicyclique ou tricyclique saturé, partiellement saturé ou aromatique, contenant 3 à 14 atomes de carbone et 0 à 3 hétéroatomes additionnels choisie parmi l'azote, l'oxygène ou le soufre ;
aryle représente un groupe aromatique monocyclique, polycyclique, biaryle et hétérocyclique non substitué, mono-, di- ou trisubstitué, lié d'une manière covalente à une position quelconque du noyau, capable de former une liaison covalente stable ;
à la condition que, quand A, avec la double liaison de la Formule 1, est le radical 4,5,6,7-tétrahydro-thiéno[2,3-c]pyridyle et que R₁ est le groupe cyano, alors R₂ ne soit pas COR₅, où R₅ est un groupe hydroxy, alkyloxy en C₁-C₆, phényloxy ou benzyloxy.

2. Composé selon la revendication 1, dans lequel A est le radical 4,5,6,7-tétrahydro-thiéno[2,3-b]pyridyle.

3. Composé selon la revendication 2, dans lequel R₁ et R₂ sont COR₅ et R₄ est un atome d'hydrogène, R₅ étant tel qus défini ci-dessus.

4. Composé selon la revendication 2, dans lequel R₁ est le radical 5-tétrazolyle et R₂ est COR₅, R₅ étant tel que défini ci-dessus.

5. Composé selon la revendication 2, dans lequel R₁ et R₂ sont COOH et R₄ est un atome d'hydrogène.

6. Composé selon la revendication 1, dans lequel A est le radical 4,5,6,7-tétrahydro-thiéno[2,3-c]pyridyle.

7. Composé selon la revendication 6, dans lequel R₁ et R₂ sont COR₅ et R₄ est un atome d'hydrogène, R₅ étant tel que défini ci-dessus.

8. Composé selon la revendication 6, dans lequel R₁ est le radical 5-tétrazolyle et R₂ est COR₅, R₅ étant tel que défini ci-dessus.

9. Composé selon la revendication 6, dans lequel R₁ et R₂ sont COOH et R₄ est un atome d'hydrogène.

10. Composé selon la revendication 1, dans lequel A est le radical 4,5,6,7-tétrahydro-thiéno[3,2-c]pyridyle.

11. Composé selon la revendication 10, dans lequel R₁ et R₂ sont COR₅ et R₄ est un atome d'hydrogène, R₅ étant tel que défini ci-dessus.

12. Composé selon la revendication 10, dans lequel R₁ est le radical 5-tétrazolyle et R₂ est COR₅, R₅ étant tel que défini ci-dessus.

13. Composé selon la revendication 10, dans lequel R₁ et R₂ sont COOH et R₄ est un atome d'hydrogène.

14. Composé selon la revendication 1, dans lequel A est le radical 4,5,6,7-tétrahydro-thiéno[3,2-b]pyridyle.

15. Composé selon la revendication 14, dans lequel R₁ et R₂ sont COR₅ et R₄ est un atome d'hydrogène, R₅ étant tel que défini ci-dessus.

16. Composé selon la revendication 14, dans lequel R₁ est le radical 5-tétrazolyle et R₂ est COR₅, R₅ étant tel que défini ci-dessus.

17. Composé selon la revendication 14, dans lequel R₁ et R₂ sont COOH et R₄ est un atome d'hydrogène.

18. Composé selon la revendication 1, dans lequel A est le radical 4,5,6,7-tétrahydro-4,7-éthano-thiéno[2,3-b]pyridyle.

19. Composé selon la revendication 18, dans lequel R₁ et R₂ sont COR₅ et R₄ est un atome d'hydrogène, R₅ étant tel que défini ci-dessus.

20. , Composé selon la revendication 18, dans lequel R₁ est le radical 5-tétrazolyle et R₂ est COR₅, R₅ étant tel que défini ci-dessus.

21. composé selon la revendication 18, dans lequel R₁ et R₂ sont COOH et R₄ est un atome d'hydrogène.

22. composé choisi parmi les composés suivants :
acide 6-benzoyl-2-(oxalylamino)-4,5,6,7-tétrahydro-thiéno[2,3-c]pyridine-3-carboxylique ;
acide 6-benzyl-2-(oxalylamino)-4,5,6,7-tétrahydro-thiéno[2,3-c]pyridine-3-carboxylique ;
acide 6-méthyl-2-(oxalylamino)-4,5,6,7-tétrahydro-thiéno[2,3-c]pyridine-3-carboxylique ;
acide 2-(oxalylamino)-6-phénéthyl-4,5,6,7-tétrahydro-thiéno[2,3-c]pyridine-3-carboxylique ;
acide 5-benzoyl-2-(oxalylamino)-4,5,6,7-tétrahydro-thiéno[2,3-c]pyridine-3-carboxylique ;
acide 5-benzyl-2-(oxalylamino)-4,5,6,7-tétrahydro-thiéno[2,3-c]pyridine-3-carboxylique ;
acide 5-méthyl-2-(oxalylamino)-4,5,6,7-tétrahydro-thiéno[2,3-c]pyridine-3-carboxylique ;
acide 2-(oxalylamino)-5-phénéthyl-4,5,6,7-tétrahydro-thiéno[2,3-c]pyridine-3-carboxylique ;
acide 2-(oxalylamino)-4,5,6,7-tétrahydro-4,7-éthano-thiéno[2,3-b]pyridine-3-carboxylique
acide 2-(oxalylamino)-6-pyridine-2-ylméthyl-4,5,6,7-tétrahydro-thiéno[2,3-c]pyridine-3-carboxylique ;
acide 2-(oxalylamino)-4,5,6,7-tétrahydro-thiéno[2,3-c]pyridine-3-carboxylique ;
acide 6-(3-méthoxy-benzyl)-2-(oxalylamino)-4,5,6,7-tétrahyrdro-thiéno[2,3-c]pyridine-3-carboxylique ;
acide 2-(oxalylamino)-6-pyridine-3-ylméthyl-4,5,6,7-tétrahydro-thiéno[2,3-c]pyridine-3-carboxylique ;
acide 2-(oxalylamino)-6-pyridine-4-ylméthyl-4,5,6,7-tétrahydro-thiéno[2,3-c]pyridine-3-carboxylique ;
aride 2-(oxalylamino)-6-quinoléine-2-ylméthyl-4,5,6,7-tétrahydro-thiéno[2,3-c]pyridine-3-carboxylique ;
acide 6-acétyl-2-(oxalylamino)-4,5,6,7-tétrahydro-thiéno[2,3-c]pyridine-3-carboxylique ;
acide 2-(oxalylamino)-6-phénylcarbamoylméthyl-4,5,6,7-tétrahydro-thiéno[2,3-c]pyridine-3,-carboxylique ;
ou l'un de ses sels acceptables d'un point de vue pharmaceutique.

23. Composés selon l'une quelconque des revendications précédentes, qui agissent comme des inhibiteurs ou des modulateurs des protéine-tyrosine-phosphatases.

24. Composition pharmaceutique convenant au traitement du diabète de type I, du diabète de type II, d'une dégradation de la tolérance au glucose, de la résistance à l'insuline ou de l'obésité, comprenant un composé selon l'une quelconque des revendications 1 à 22 ou un sel acceptable d'un point de vue pharmaceutique de ces derniers avec un acide ou une base acceptable d'un point de vue pharmaceutique, ou tout isomère optique ou mélange d'isomères optiques, y compris un mélange racémique, ou toutes formes tautomères, avec un ou plusieurs excipients ou diluants acceptables d'un point de vue pharmaceutique.
